(19) Europäisches Patentamt
European Patent Office
Office européen des brevets

(11) EP 4 223 868 A1

(12) EUROPEAN PATENT APPLICATION
published in accordance with Art. 153(4) EPC

(43) Date of publication:
**09.08.2023 Bulletin 2023/32**

(21) Application number: **21875721.9**

(22) Date of filing: **29.09.2021**

(51) International Patent Classification (IPC):
***C12N 1/20*** (2006.01) ***A61K 35/741*** (2015.01)
***A61K 39/395*** (2006.01) ***A61K 45/00*** (2006.01)
***A61P 35/00*** (2006.01) ***A61P 35/02*** (2006.01)
***A61P 35/04*** (2006.01) ***A61P 37/04*** (2006.01)
***A61P 43/00*** (2006.01) ***C12N 15/11*** (2006.01)
***C12Q 1/6869*** (2018.01)

(52) Cooperative Patent Classification (CPC):
**A61K 35/741; A61K 39/395; A61K 45/00; A61P 35/00; A61P 35/02; A61P 35/04; A61P 37/04; A61P 43/00; C12N 1/20; C12N 15/11; C12Q 1/6869**

(86) International application number:
**PCT/JP2021/035941**

(87) International publication number:
**WO 2022/071423 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2020 JP 2020165470**

(71) Applicants:
- **National Cancer Center**
**Tokyo 104-0045 (JP)**
- **RIKEN**
**Wako-shi, Saitama 351-0198 (JP)**

(72) Inventors:
- **NISHIKAWA, Hiroyoshi**
**Kashiwa-shi, Chiba 277-8577 (JP)**
- **FUKUOKA, Shota**
**Kashiwa-shi, Chiba 277-8577 (JP)**
- **BENNO, Yoshimi**
**Wako-shi, Saitama 351-0198 (JP)**
- **SHIGENO, Yuko**
**Wako-shi, Saitama 351-0198 (JP)**

(74) Representative: **Plasseraud IP**
**66, rue de la Chaussée d'Antin**
**75440 Paris Cedex 09 (FR)**

Remarks:
The complete document including Reference Table(s) and the Sequence Listing(s) can be downloaded from the EPO website

# (54) ENHANCEMENT OF ANTITUMOR EFFECT OF IMMUNE CHECKPOINT INHIBITOR THROUGH ADMINISTRATION OF INTESTINAL RUMINOCOCCACEAE BACTERIUM

(57) To provide a pharmaceutical composition capable of enhancing the effect of an immune checkpoint inhibitor against tumor or cancer in a subject. Provided is a pharmaceutical composition that comprises bacterial cells, a culture supernatant, a metabolite, and/or a bacterial cell extract of *Ruminococcaceae* enterobacterium, and is administered in combination with an immune checkpoint inhibitor.

EP 4 223 868 A1

Fig. 1

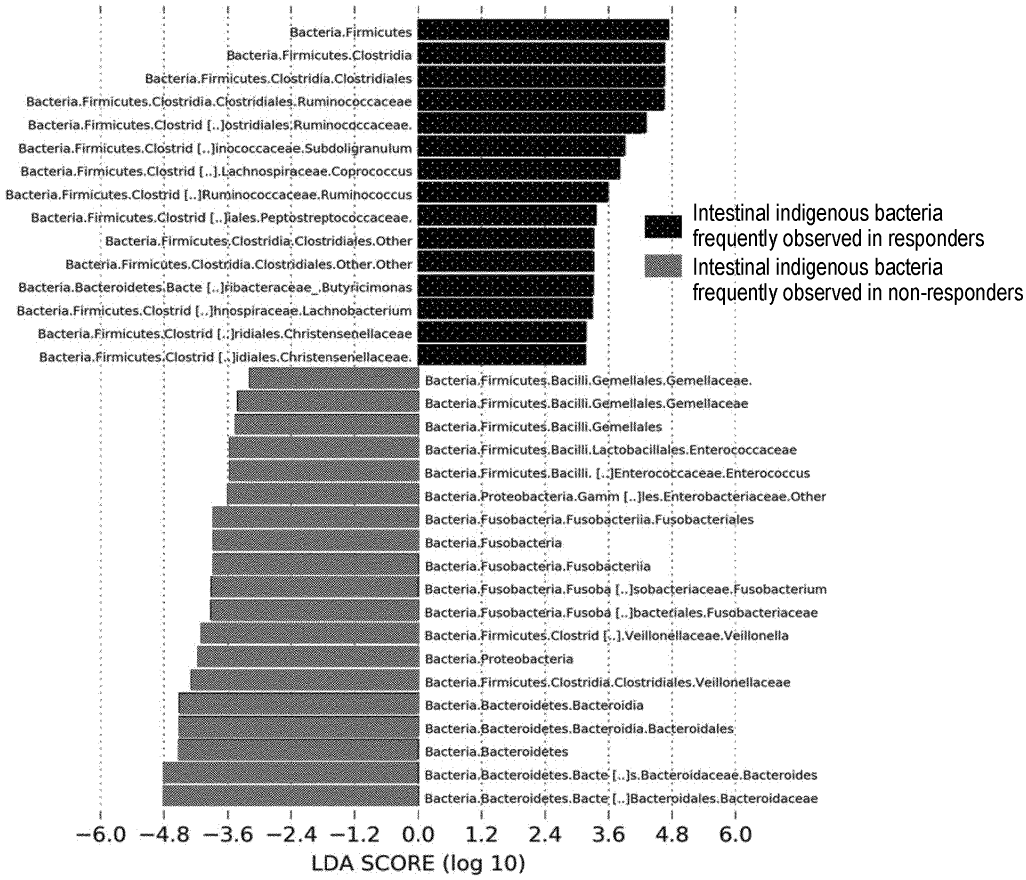
Bacteria.Firmicutes
Bacteria.Firmicutes.Clostridia
Bacteria.Firmicutes.Clostridia.Clostridiales
Bacteria.Firmicutes.Clostridia.Clostridiales.Ruminococcaceae
Bacteria.Firmicutes.Clostrid [..]ostridiales.Ruminococcaceae.
Bacteria.Firmicutes.Clostrid [..]inococcaceae.Subdoligranulum
Bacteria.Firmicutes.Clostrid [..].Lachnospiraceae.Coprococcus
Bacteria.Firmicutes.Clostrid [..]Ruminococcaceae.Ruminococcus
Bacteria.Firmicutes.Clostrid [..]iales.Peptostreptococcaceae.
Bacteria.Firmicutes.Clostridia.Clostridiales.Other
Bacteria.Firmicutes.Clostridia.Clostridiales.Other.Other
Bacteria.Bacteroidetes.Bacte [..]ribacteraceae_.Butyricimonas
Bacteria.Firmicutes.Clostrid [..]hnospiraceae.Lachnobacterium
Bacteria.Firmicutes.Clostrid [..]ridiales.Christensenellaceae
Bacteria.Firmicutes.Clostrid [..]idiales.Christensenellaceae.
Bacteria.Firmicutes.Bacilli.Gemellales.Gemellaceae.
Bacteria.Firmicutes.Bacilli.Gemellales.Gemellaceae
Bacteria.Firmicutes.Bacilli.Gemellales
Bacteria.Firmicutes.Bacilli.Lactobacillales.Enterococcaceae
Bacteria.Firmicutes.Bacilli. [..]Enterococcaceae.Enterococcus
Bacteria.Proteobacteria.Gamm [..]les.Enterobacteriaceae.Other
Bacteria.Fusobacteria.Fusobacteriia.Fusobacteriales
Bacteria.Fusobacteria
Bacteria.Fusobacteria.Fusobacteriia
Bacteria.Fusobacteria.Fusoba [..]sobacteriaceae.Fusobacterium
Bacteria.Fusobacteria.Fusoba [..]bacteriales.Fusobacteriaceae
Bacteria.Firmicutes.Clostrid [..].Veillonellaceae.Veillonella
Bacteria.Proteobacteria
Bacteria.Firmicutes.Clostridia.Clostridiales.Veillonellaceae
Bacteria.Bacteroidetes.Bacteroidia
Bacteria.Bacteroidetes.Bacteroidia.Bacteroidales
Bacteria.Bacteroidetes
Bacteria.Bacteroidetes.Bacte [..]s.Bacteroidaceae.Bacteroides
Bacteria.Bacteroidetes.Bacte [..]Bacteroidales.Bacteroidaceae
Intestinal indigenous bacteria frequently observed in responders
Intestinal indigenous bacteria frequently observed in non-responders
−6.0 −4.8 −3.6 −2.4 −1.2 0.0 1.2 2.4 3.6 4.8 6.0
LDA SCORE (log 10)

## Description

### TECHNICAL FIELD

The present invention relates to a pharmaceutical composition for enhancing the effect of an immune checkpoint inhibitor against tumor or cancer in a subject. The present invention relates particularly to a pharmaceutical composition containing bacterial cells, a culture supernatant, a metabolite, and/or a bacterial cell extract of *Ruminococcaceae* enterobacterium, which composition is administered in combination with an immune checkpoint inhibitor.

### BACKGROUND ART

Immune checkpoint inhibitors elicit marked clinical benefits on cancers and tumors during treatment using an immunosuppressive mechanism as a target. Immune checkpoint inhibitors have been approved for several types of tumor or cancer, including malignant melanoma, lung cancer, renal cell carcinoma, head and neck cancer, and gastric cancer. However, it is said that the therapeutic effect of an immune checkpoint inhibitor alone has not yet been satisfactory.
Different immunosuppressive inhibitory drugs may be used in combination or existing approved drugs may be used in combination therewith to simultaneously disable multiple immunosuppressive mechanisms. This approach, in which anti-tumor immunities are further activated and the efficacy is thus enhanced, has been thereby proposed (PTL1). In fact, several clinical trials are on-going. However, it is currently difficult to say that any sufficient effect has been obtained.
It is known that an immunosuppressive network based on immune checkpoint molecules and regulatory T cells is established in the cancer microenvironment and induces immune tolerance (NPL1). It has been successively reported that intestinal indigenous bacteria affect these immune responses (NPL2 and NPL3).

### CITATION LIST

### PATENT LITERATURE

PTL1: Japanese Translation of PCT International Application Publication No. 2015-518826

### NON PATENT LITERATURE

NPL1: Nature Reviews Cancer, 12, 252-264 (2012)
NPL2: Science, 359, 91-97 (2018)
NPL3: Science, 359, 97-103 (2018)

### SUMMARY OF INVENTION

### TECHNICAL PROBLEM

The purpose of the present invention is to provide a pharmaceutical composition capable of enhancing the effect of an immune checkpoint inhibitor against a tumor or cancer in a subject.

### SOLUTION TO PROBLEM

The present inventors analyzed enterobacteria of patients with gastric/lung cancer who had been treated with an immune checkpoint inhibitor and have found that responders to the immune checkpoint inhibitor often had bacteria of an unclassified genus in the family *Ruminococcaceae.* Further, the present inventors isolated and cultured bacteria from the intestinal contents of the responders so as to investigate how specific bacteria affected anti-tumor immunity. The isolated bacteria were administered to mice in which native intestinal bacteria had been reduced by antimicrobial administration to examine the effects of the bacteria in combination with immune checkpoint inhibitors. As a result, *Ruminococcaceae* YB328, which has 16S rRNA gene with the nucleotide sequence set forth in SEQ ID NO: 1, has been found to enhance the anti-tumor effect of the immune checkpoint inhibitor. The present invention has then been completed.
Specifically, the invention pertains to, but is not limited to, the following items.

[1] A method of isolating a *Ruminococcaceae* enterobacterium, comprising the steps of:

(i) producing a diluted intestinal content liquid by serial dilution, using an anaerobic diluent, of intestinal contents obtained from a mammal that has received an immune checkpoint inhibitor and that has been evaluated as PR (partial response) or better or SD (stable disease) for six months or longer by CT imaging after administration;
(ii) inoculating a portion of the diluted intestinal content liquid into a solid medium for culturing under anaerobic conditions to form, on the solid medium, a colony(s) derived from a single clone of microorganisms contained in the diluted intestinal content liquid;
(iii) confirming whether or not a bacterium contained in the colony has 16S rRNA gene with 95% or higher sequence identity to a nucleotide sequence set forth in SEQ ID NO: 1; and
(iv) obtaining the bacterium confirmed to have the 16S rRNA gene with 95% or higher sequence identity to the nucleotide sequence set forth in SEQ ID NO: 1.

[2] The isolation method according to [1], wherein the immune checkpoint inhibitor is an inhibitor for any of immune checkpoint molecules selected from the group consisting of PD-1, CTLA-4, TIM-3, BTLA, LAG-3, A2aR, KIR, VISTA, TIGIT, PD-L1 PD-L2, CD80, CD86, GAL-9, HVEM, CD160, MHC class II, B7-H3, B7-H4, B7-H5, B7-H6, and B7-H7, or a combination of two or more different inhibitors therefor.
[3] The isolation method according to [2], wherein the immune checkpoint inhibitor is selected from an antibody against the immune checkpoint molecule, an antigen-binding fragment of the antibody, or a combination thereof.
[4] The isolation method according to [3], wherein the immune checkpoint inhibitor is selected from the group consisting of nivolumab, pembrolizumab, cemiplimab, avelumab, atezolizumab, and durvalumab.
[5] The isolation method according to any one of [1] to [4], wherein the mammal is a human.
[6] A method for producing a pharmaceutical composition, comprising the step of making a pharmaceutical composition by blending bacterial cells, a culture supernatant, a metabolite, and/or a bacterial cell extract of *Ruminococcaceae* enterobacterium isolated by the isolation method according to any one of [1] to [5].
[7] The production method according to [6], wherein the pharmaceutical composition is a pharmaceutical composition administered in combination with an immune checkpoint inhibitor.
[8] A pharmaceutical composition produced by the production method according to [6] or [7].
[9] A pharmaceutical composition comprising bacterial cells, a culture supernatant, a metabolite, and/or a bacterial cell extract of *Ruminococcaceae* enterobacterium, wherein the composition is administered in combination with an immune checkpoint inhibitor.
[10] The pharmaceutical composition according to [9], comprising viable cells of *Ruminococcaceae* enterobacterium.
[11] The pharmaceutical composition according to [9] or [10], wherein the *Ruminococcaceae* enterobacterium is a bacterium having 16S rRNA gene with 95% or higher identity to a nucleotide sequence set forth in SEQ ID NO: 1.
[12] The pharmaceutical composition according to any one of [8] to [11], wherein the immune checkpoint inhibitor is an inhibitor for any of immune checkpoint molecules selected from the group consisting of PD-1, CTLA-4, TIM-3, BTLA, LAG-3, A2aR, KIR, VISTA, TIGIT, PD-L1 PD-L2, CD80, CD86, GAL-9, HVEM, CD160, MHC class II, B7-H3, B7-H4, B7-H5, B7-H6, and B7-H7, or a combination of two or more different inhibitors therefor.
[13] The pharmaceutical composition according to [12], wherein the immune checkpoint inhibitor is selected from an antibody against the immune checkpoint molecule, an antigen-binding fragment of the antibody, or a combination thereof.
[14] The pharmaceutical composition according to [13], wherein the immune checkpoint inhibitor is selected from the group consisting of nivolumab, pembrolizumab, cemiplimab, avelumab, atezolizumab, and durvalumab.
[15] The pharmaceutical composition according to any one of [8] to [14], which is administered by oral, tubal, or enema administration.
[16] The pharmaceutical composition according to any one of [8] to [15], wherein the bacterial cells, the culture supernatant, the metabolite, and/or the bacterial cell extract of *Ruminococcaceae* enterobacterium is administered simultaneously with the immune checkpoint inhibitor.
[17] The pharmaceutical composition according to [16], comprising the immune checkpoint inhibitor and the bacterial cells, the culture supernatant, the metabolite, and/or the bacterial cell extract of *Ruminococcaceae* enterobacterium.
[18] The pharmaceutical composition according to any one of [8] to [15], wherein the bacterial cells, the culture supernatant, the metabolite, and/or the bacterial cell extract of *Ruminococcaceae* enterobacterium and the immune checkpoint inhibitor are administered separately.
[19] The pharmaceutical composition according to [18], wherein before administration of the immune checkpoint inhibitor, the bacterial cells, the culture supernatant, the metabolite, and/or the bacterial cell extract of *Ruminococcaceae* enterobacterium is administered.
[20] The pharmaceutical composition according to [18], wherein after administration of the immune checkpoint inhibitor, the bacterial cells, the culture supernatant, the metabolite, and/or the bacterial cell extract of *Ruminococcaceae* enterobacterium is administered.
[21] The pharmaceutical composition according to any one of [8] to [20] for activating CD8-positive T cells in a subject.

[22] The pharmaceutical composition according to any one of [8] to [21] for enhancing the immune response against tumor or cancer in a subject with a tumor or cancer.

[23] The pharmaceutical composition according to [22], wherein the effect of enhancing the immune response against tumor or cancer is greater than when the immune checkpoint inhibitor is administered alone.

[24] The pharmaceutical composition according to any one of [8] to [23] for treating a tumor or cancer in a subject.

[25] The pharmaceutical composition according to [24], wherein the treatment is to eliminate, reduce, or stabilize the tumor or cancer.

[26] The pharmaceutical composition according to [25], wherein the effect of eliminating, reducing, or stabilizing the tumor or cancer is greater than when the immune checkpoint inhibitor is administered alone.

[27] The pharmaceutical composition according to any one of [8] to [25] for suppressing recurrence or metastasis of a tumor or cancer in a subject.

[28] The pharmaceutical composition according to [27], wherein the effect of suppressing the recurrence or metastasis of the tumor or cancer is greater than when the immune checkpoint inhibitor is administered alone.

[29] The pharmaceutical composition according to any one of [22] to [28], wherein the pharmaceutical composition is further administered in combination with at least one therapy selected from the group consisting of surgery, chemotherapy, and radiation therapy.

[30] The pharmaceutical composition according to any one of [22] to [29], wherein the tumor or cancer is selected from the group consisting of malignant pleural mesothelioma, malignant peritoneal mesothelioma, malignant melanoma, malignant lymphoma, brain tumor, glioma, neuroblastoma, thymoma, gastrointestinal stromal tumor, neuroendocrine tumor, testicular tumor, soft tissue sarcoma, nephroblastoma, hepatoblastoma, germ cell tumor, retinoblastoma, osteosarcoma, Ewing sarcoma, rhabdomyosarcoma, acute myelogenous leukemia, acute lymphocytic leukemia, chronic myelogenous leukemia, chronic lymphocytic leukemia, myelodysplastic syndrome, adult T-cell leukemia, multiple myeloma, oropharyngeal cancer, laryngeal cancer, tongue cancer, nasal cancer, sinus cancer, thyroid cancer, parotid cancer, submandibular gland cancer, auditory cancer, lung cancer, breast cancer, thymic cancer, esophageal cancer, gastric cancer, colon cancer, small intestine cancer, hepatocellular carcinoma, bile duct cancer, gallbladder cancer, pancreatic cancer, renal cell carcinoma, renal pelvis and ureter cancer, bladder cancer, ureteral duct cancer, adrenal carcinoma, peritoneal carcinoma, prostate cancer, cervical cancer, uterine cancer, ovarian cancer, vaginal cancer, vulvar cancer, basal cell carcinoma, spinous cell carcinoma, neuroendocrine cancer, Kaposi sarcoma, and cancer of unknown primary origin.

[31] The pharmaceutical composition according to any one of [8] to [20] for increasing diversity of intestinal indigenous bacteria in a mammal when compared to that before administration.

[32] A method of enhancing an immune response against a tumor or cancer, comprising administering to a subject with a tumor or cancer an effective amount of an immune checkpoint inhibitor in combination with an effective amount of a pharmaceutical composition containing bacterial cells, a culture supernatant, a metabolite, and/or a bacterial cell extract of *Ruminococcaceae* enterobacterium.

[33] The method according to [32], wherein a larger anti-tumor or -cancer immune response-enhancing effect is exerted than when the immune checkpoint inhibitor is administered alone.

[34] A method of treating a tumor or cancer, comprising administering to a subject with the tumor or cancer an effective amount of an immune checkpoint inhibitor in combination with an effective amount of a pharmaceutical composition containing bacterial cells, a culture supernatant, a metabolite, and/or a bacterial cell extract of *Ruminococcaceae* enterobacterium.

[35] The method according to [34], wherein the treatment is to eliminate, reduce, or stabilize the tumor or cancer.

[36] The method according to [35], wherein the effect of eliminating, reducing, or stabilizing the tumor or cancer is greater than when the immune checkpoint inhibitor is administered alone.

[37] A method of suppressing recurrence or metastasis of a tumor or cancer in a subject, comprising administering to the subject with the tumor or cancer an effective amount of an immune checkpoint inhibitor in combination with an effective amount of a pharmaceutical composition containing bacterial cells, a culture supernatant, a metabolite, and/or a bacterial cell extract of *Ruminococcaceae* enterobacterium.

[38] The method according to [37], wherein the effect of suppressing the recurrence or metastasis of the tumor or cancer is greater than when the immune checkpoint inhibitor is administered alone.

[39] The method according to any one of [32] to [38], wherein the bacterial cells, the culture supernatant, the metabolite, and/or the bacterial cell extract of *Ruminococcaceae* enterobacterium is administered simultaneously with the immune checkpoint inhibitor.

[40] The method according to any one of [32] to [38], wherein the bacterial cells, the culture supernatant, the metabolite, and/or the bacterial cell extract of *Ruminococcaceae* enterobacterium and the immune checkpoint inhibitor are administered separately.

[41] The method according to [40], wherein before administration of the immune checkpoint inhibitor, the bacterial cells, the culture supernatant, the metabolite, and/or the bacterial cell extract of *Ruminococcaceae* enterobacterium is administered.

[42] The pharmaceutical composition according to [40], wherein after administration of the immune checkpoint inhibitor, the bacterial cells, the culture supernatant, the metabolite, and/or the bacterial cell extract of *Ruminococcaceae* enterobacterium is administered.

[43] The method according to any one of [32] to [42], wherein the method is further performed in combination with at least one therapy selected from the group consisting of surgery, chemotherapy, and radiation therapy.

[44] The method according to any one of [32] to [43], wherein the tumor or cancer is selected from the group consisting of malignant pleural mesothelioma, malignant peritoneal mesothelioma, malignant melanoma, malignant lymphoma, brain tumor, glioma, neuroblastoma, thymoma, gastrointestinal stromal tumor, neuroendocrine tumor, testicular tumor, soft tissue sarcoma, nephroblastoma, hepatoblastoma, germ cell tumor, retinoblastoma, osteosarcoma, Ewing sarcoma, rhabdomyosarcoma, acute myelogenous leukemia, acute lymphocytic leukemia, chronic myelogenous leukemia, chronic lymphocytic leukemia, myelodysplastic syndrome, adult T-cell leukemia, multiple myeloma, oropharyngeal cancer, laryngeal cancer, tongue cancer, nasal cancer, sinus cancer, thyroid cancer, parotid cancer, submandibular gland cancer, auditory cancer, lung cancer, breast cancer, thymic cancer, esophageal cancer, gastric cancer, colon cancer, small intestine cancer, hepatocellular carcinoma, bile duct cancer, gallbladder cancer, pancreatic cancer, renal cell carcinoma, renal pelvis and ureter cancer, bladder cancer, ureteral duct cancer, adrenal carcinoma, peritoneal carcinoma, prostate cancer, cervical cancer, uterine cancer, ovarian cancer, vaginal cancer, vulvar cancer, basal cell carcinoma, spinous cell carcinoma, neuroendocrine cancer, Kaposi sarcoma, and cancer of unknown primary origin.

[45] A method of increasing the number of intestinal indigenous bacteria in a mammal when compared to that before administration, comprising administering to the mammal an effective amount of a pharmaceutical composition containing bacterial cells, a culture supernatant, a metabolite, and/or a bacterial cell extract of *Ruminococcaceae* enterobacterium.

[46] A pharmaceutical composition for inducing dendritic cell progenitors to type 1 dendritic cells, comprising agonists for multiple TLRs other than TLR4.

[47] The pharmaceutical composition according to [46], wherein the multiple TLRs are TLR1, TLR3, TLR5, TLR7, and TLR9.

[48] The pharmaceutical composition according to [47], wherein the multiple TLRs are TLR5, TLR7, and TLR9.

[49] The pharmaceutical composition according to [48], wherein the agonist is a combination of flagellin, R848 (resiquimod), and CpG-ODN.

[50] The pharmaceutical composition according to any one of [46] to [48], wherein the agonist is bacterial cells, a culture supernatant, a metabolite, and/or a bacterial cell extract of *Ruminococcaceae* enterobacterium.

[51] The pharmaceutical composition according to any one of [46] to [50] for treating a tumor or cancer in a subject.

[52] A method of inducing dendritic cell progenitors to type 1 dendritic cells, comprising bringing agonists for multiple TLRs other than TLR4 in contact with the dendritic cell progenitors.

[53] The method according to [52], wherein the method is performed *in vitro.*

[54] The method according to [52] or [53], wherein the multiple TLRs are TLR5, TLR7, and TLR9.

[55] The method according to [54], wherein the agonist is a combination of flagellin, R848 (resiquimod), and CpG-ODN.

[56] The method according to any one of [52] to [55], wherein the agonist is bacterial cells, a culture supernatant, a metabolite, and/or a bacterial cell extract of *Ruminococcaceae* enterobacterium.

[57] Type 1 dendritic cells induced by the method according to any one of [52] to [56].

[58] A pharmaceutical composition comprising the type 1 dendritic cells according to [57] for treating a tumor or cancer in a subject.

[59] A pharmaceutical composition comprising the type 1 dendritic cells according to [57] for enhancing an immune response against a tumor or cancer in a subject with a tumor or cancer.

## ADVANTAGEOUS EFFECTS OF INVENTION

The present invention allows to enhance the effect of an immune checkpoint inhibitor against a tumor or cancer in a subject.

## BRIEF DESCRIPTION OF DRAWINGS

[Fig. 1] Fig. 1 shows the results of LEfSe analysis in which intestinal indigenous bacteria are compared between responders and non-responders to an immune checkpoint inhibitor.

[Fig. 2] Fig. 2 shows the results of comparing the progression-free survival between patients with a high median percentage of and patients with a low median percentage of each bacterial group in the intestinal indigenous bacteria.
[Fig. 3] Fig. 3 shows the results of investigating the effects of an immune checkpoint inhibitor in a mouse tumor model in which intestinal contents derived from either responders or non-responders to an immune checkpoint inhibitor were transplanted.
[Fig. 4] Fig. 4 shows the results of investigating the effects of an immune checkpoint inhibitor in an antibiotic-treated mouse tumor model transplanted with intestinal contents derived from responders to the immune checkpoint inhibitor.
[Fig. 5] Fig. 5 shows the results of phylogenetic analysis of 16S rRNA gene sequences of bacteria in intestinal contents derived from responders to an immune checkpoint inhibitor.
[Fig. 6] Fig. 6 shows the results of investigating the effects of an immune checkpoint inhibitor in an antibiotic-treated mouse tumor model transplanted with each bacterium species.
[Fig. 7] Fig. 7 shows the results of investigating the effects of an immune checkpoint inhibitor in an antibiotic-treated mouse tumor model transplanted with *B. vulgatus* or *Ruminococcaceae* YB328.
[Fig. 8] Fig. 8 shows the results of flow cytometry measuring dendritic cell maturation markers on dendritic cells co-cultured with *Ruminococcaceae* YB328 or *B. vulgatus* or vehicle.
[Fig. 9] Fig. 9 shows the results of measuring an activation marker (IFN-$\gamma$) for $CD8^+$ T cells when dendritic cells after co-cultured with bacteria were co-cultured with $CD8^+$ T cells derived from OT-I mice and stimulated with 1 nM or 100 nM of N4 peptide.
[Fig. 10] Fig. 10 shows the results of measuring TCR signaling (ZAP70) and CD28 signaling (Erk) when dendritic cells after co-cultured with bacteria were co-cultured with $CD8^+$ T cells derived from OT-I mice and stimulated with N4 peptide or Q4H7 peptide.
[Fig. 11] Fig. 11 shows the results of investigating how stimulation of N4 peptide with different concentrations (0 nM, 1 nM, 10 nM, or 100 nM) affected TCR signaling (ZAP70 (pZAP70)) and CD28 signaling (Erk (pErk), Akt (pAkt), S6 (pS6)) when dendritic cells co-cultured with *Ruminococcaceae* YB328 were co-cultured with $CD8^+$ T cells or when dendritic cells co-cultured with *B. vulgatus* were co-cultured with $CD8^+$ T cells, respectively.
[Fig. 12] Fig. 12 shows the results of investigating the effects of an immune checkpoint inhibitor in an antibiotic-treated mouse tumor model in which intestinal contents derived from responders or non-responders to the immune checkpoint inhibitor were transplanted, and *Ruminococcaceae* YB328 alone or *B. vulgatus* bacterium alone was orally administered.
[Fig. 13] Fig. 13 shows the results of conducting meta-analysis based on 16S rRNA gene in intestinal contents of mice as collected either after transplantation of intestinal contents or after administration of bacterium alone, and comparing the diversity of bacterial flora in intestinal contents in each case.
[Fig. 14] Fig. 14 shows the results of performing transcriptome analysis after mouse bone marrow-derived dendritic cells were co-cultured with *Ruminococcaceae* YB328 or *B. vulgatus* or LPS or vehicle (PBS) and RNA was then extracted from the dendritic cells.
[Fig. 15] Fig. 15 shows the results of FACS analysis of each tissue (lymph node near the tumor, mucosa lamina propria, or intestinal peritoneal lymph node) collected from each mouse in which MC38 cultured cell line was subcutaneously transplanted and *Ruminococcaceae* YB328 or *B. vulgatus* was then orally administered.
[Fig. 16] Fig. 16 shows the results of FACS analysis of a tumor collected from each mouse in which MC38 cultured cell line was subcutaneously transplanted and *Ruminococcaceae* YB328 or *B. vulgatus* was then orally administered.
[Fig. 17] Fig. 17 shows the results of FACS analysis of IRF8 expression after bone marrow-derived dendritic cell progenitors were co-cultured with FLT3L and *Ruminococcaceae* YB328 or *B. vulgatus* or LPS or PBS.
[Fig. 18] Fig. 18 shows the results of FACS analysis of p-S6K and p-STAT3 expression after mouse bone marrow-derived dendritic cells were co-cultured with *Ruminococcaceae* YB328 or *B. vulgatus* or LPS or PBS.
[Fig. 19] Fig. 19 shows the induction of dendritic cell progenitors into type 1 dendritic cells by combined TLR stimulation. A shows the results of transcriptome analysis focusing on various TLRs in dendritic cells stimulated with *Ruminococcaceae* YB328 or *B. vulgatus.* B shows the results of FACS analysis on the percentage of CD103-positive CD11b-negative dendritic cells obtained by stimulating, with *Ruminococcaceae* YB328 or vehicle, bone marrow-derived dendritic cells collected from MyD88-knockout mice. C shows the results of FACS analysis on the percentage of CD103-positive CD11b-negative dendritic cells obtained by stimulating mouse bone marrow-derived dendritic cells with each TLR5, 7, and/or 9 agonist mixture.

DESCRIPTION OF EMBODIMENTS

One aspect of the present invention provides a method of isolating a *Ruminococcaceae* enterobacterium.
The method of isolating a *Ruminococcaceae* enterobacterium according to the present invention comprises the steps of:

(i) producing a diluted intestinal content liquid by serial dilution, using an anaerobic diluent, of intestinal contents obtained from a mammal that has received an immune checkpoint inhibitor and that has been evaluated as PR (partial response) or better or SD (stable disease) for six months or longer by CT imaging after administration;
(ii) inoculating a portion of the diluted intestinal content liquid into a solid medium for culturing under anaerobic conditions to form, on the solid medium, a colony(s) derived from a single clone of microorganisms contained in the diluted intestinal content liquid;
(iii) checking whether or not a bacterium contained in the colony has 16S rRNA gene with 95% or higher sequence identity to a nucleotide sequence set forth in SEQ ID NO: 1; and
(iv) obtaining the bacterium found to have the 16S rRNA gene with 95% or higher sequence identity to the nucleotide sequence set forth in SEQ ID NO: 1.

<Method of isolating *Ruminococcaceae* enterobacterium>

A *Ruminococcaceae* enterobacterium in the present invention may be administered in combination with an immune checkpoint inhibitor, thereby allowing to enhance the effects of the immune checkpoint inhibitor against tumors or cancers in a subject. The present inventors have surprisingly found that such a *Ruminococcaceae* enterobacterium can be isolated from humans who are responders to the immune checkpoint inhibitor.

<Step of producing diluted intestinal content liquid>

The method of isolating a *Ruminococcaceae* enterobacterium according to the present invention comprises the step of (i) producing a diluted intestinal content liquid by serial dilution, using an anaerobic diluent, of intestinal contents obtained from a mammal that has received an immune checkpoint inhibitor and that has been evaluated as PR (partial response) or better or SD (stable disease) for six months or longer by CT imaging after administration.

CR (Complete Response), PR (Partial Response), SD (Stable Disease), and PD (Progressive Disease) are common criteria used in the art as indicators of tumor or cancer disappearance, reduction, or stabilization. CR is complete disappearance of tumor, PR is a reduction in the total tumor size by 30% or more, SD is a state without any change in tumor size, and PD is an increase in the total tumor size by 20% or more and an absolute value increase by 5 mm or more, or appearance of a new lesion. The tumor size can be evaluated by CT imaging. For CT imaging, RECIST ver1.1 can be used, for example.

In the method of isolating a *Ruminococcaceae* enterobacterium according to the present invention, intestinal contents are used that have been obtained from a mammal that has received an immune checkpoint inhibitor and that has been evaluated as PR (partial response) or better or SD (stable disease) for six months or longer by CT imaging after administration. As used herein, PR (partial response) or better is defined as PR (partial response) or CR (complete response).

The method of isolating a *Ruminococcaceae* enterobacterium according to the present invention comprises the step of producing a diluted intestinal content liquid by serial dilution, using an anaerobic diluent, of the intestinal contents. Any diluent that does not adversely affect the survival of anaerobic bacteria may be used as the anaerobic diluent. For example, according to the description in "The World of Enterobacteria" by Tomotari Mitsuoka (1990), Asakura Publishing Co., Ltd., anaerobic diluent (B) in the book may be used. The dilution factor may be adjusted, if appropriate, but for colony formation on a solid medium, for example, a 10-fold dilution series in the range of $10^{-6}$ to $10^{-10}$ may be made, and the dilution factor most suitable for the colony formation can be selected.

<Step of forming colonies>

The method of isolating a *Ruminococcaceae* enterobacterium according to the present invention comprises the step of inoculating a portion of the diluted intestinal content liquid into a solid medium for culturing under anaerobic conditions to form, on the solid medium, a colony/colonies derived from a single clone of microorganisms contained in the diluted intestinal content liquid.

For example, EG agar medium may be used as the solid medium. The standard composition of EG agar medium is shown below.

[Table 1]

[Table 1]

| | |
|---|---|
| Lab-Lemco powder (Oxoid, Inc.) | 2.4 g |
| Proteose Peptone No. 3 (BD-Difco, Inc.) | 10.0 g |
| Yeast extract (BD-Difco, Inc.) | 5.0 g |
| $Na_2HPO_4$ | 4.0 g |
| Glucose | 1.5 g |
| Soluble starch | 0.5 g |
| L-Cystine | 0.2 g |
| Agar | 15.0 g |
| L-Cysteine hydrochloride | 0.5 g |
| Horse blood | 50.0 mL |
| Distilled water | 950.0 mL |

The anaerobic conditions are defined as an environment where oxygen is absent and the environment is replaced by nitrogen gas, hydrogen gas, and carbon dioxide gas as a gas phase. A virtually oxygen-free environment may be achieved in an enclosed environment (e.g., an anaerobic chamber) that can maintain an atmosphere with an oxygen partial pressure low enough to allow the growth of *Ruminococcaceae* enterobacterium.

<Step of checking nucleotide sequence of 16S rRNA gene of bacterium>

The method of isolating a *Ruminococcaceae* enterobacterium according to the present invention comprises the step of checking whether or not a bacterium contained in the colony formed has 16S rRNA gene with 95% or higher sequence identity to a nucleotide sequence set forth in SEQ ID NO: 1.

The *Ruminococcaceae* enterobacterium in the present invention refers to a strictly anaerobic bacterium classified into the phylum *Firmicutes,* the class *Clostridia,* the order *Clostridia,* and the family *Ruminococcaceae.* In particular, the bacterium has 16S rRNA gene with 95% or higher, 96% or higher, 97% or higher, 98% or higher, or 99% or higher sequence identity to the nucleotide sequence set forth in SEQ ID NO: 1.

The % identity between two gene nucleotide sequences may be determined by visual inspection and mathematical calculation. More preferably, for this comparison, sequence information is compared using a computer program. A typical, preferred computer program is the Wisconsin Package, version 10.0, program "GAP" from the Genetics Computer Group (GCG; Madison, Wisconsin) (Devereux, et al., 1984, Nucl. Acids Res, 12: 387). It is possible to use other sequence comparison programs used by those skilled in the art (e.g., BLASTN program, version 2.2.7, available through use of the U.S. National Library of Medicine website: http://www.ncbi.nlm.nih.gov/blast/bl2seq/bls.html, or the UW-BLAST 2.0 algorithm). Standard default parameters of UW-BLAST 2.0 may be set using those described in the following internet site: http://blast.wustl.edu.

The method includes checking whether or not a bacterium contained in the colony formed has 16S rRNA gene with 95% or higher sequence identity to the nucleotide sequence set forth in SEQ ID NO: 1. The method is, for example, to determine the nucleotide sequence of 16S rRNA gene of the bacterium included in each colony and compare it with the nucleotide sequence of SEQ ID NO: 1. The 16S rRNA gene of the bacterium may be amplified by PCR using known primers and sequenced by a standard sequencing method.

<Step of obtaining bacterium>

The method of isolating a *Ruminococcaceae* enterobacterium according to the present invention comprises the step of (iv) obtaining the bacterium found to have the 16S rRNA gene with 95% or higher sequence identity to the nucleotide sequence set forth in SEQ ID NO: 1.

The *Ruminococcaceae* enterobacterium, which has been found to have 16S rRNA gene with 95% or higher sequence identity to the nucleotide sequence set forth in SEQ ID NO: 1 by the above method, may be inoculated, from the colony, into a liquid medium and further cultured in a large scale for use. For example, it is possible to use, as the liquid medium, a liquid medium free of agar in the above EG agar medium composition (hereinafter, simply sometimes referred to as EG medium).

<Step of confirming the effect of enhancing the effect of immune checkpoint inhibitor against tumor or cancer>

The method of isolating a *Ruminococcaceae* enterobacterium according to the present invention optionally includes, if necessary, the step of confirming whether the obtained *Ruminococcaceae* enterobacterium has an effect of enhancing the effect of the immune checkpoint inhibitor against tumor or cancer. For instance, the obtained *Ruminococcaceae* enterobacterium and an immune checkpoint inhibitor may be used in combination. The combination may be administered to a mammal such as a mouse, rat, or human with a tumor or cancer. This case may be compared to the case where the immune checkpoint inhibitor is administered alone to check whether the effect of the immune checkpoint inhibitor is enhanced. Specifically, for example, the tumor or cancer may be eliminated, reduced in the size, or stabilized without any size change in the case where the obtained *Ruminococcaceae* enterobacterium and an immune checkpoint inhibitor are used in combination and administered when compared to the case where the immune checkpoint inhibitor is administered alone. The former case can be evaluated such that the effect of the immune checkpoint inhibitor is enhanced.

<*Ruminococcaceae* enterobacterium for pharmaceutical composition>

One aspect of the present invention provides a pharmaceutical composition comprising bacterial cells, a culture supernatant, a metabolite, and/or a bacterial cell extract of *Ruminococcaceae* enterobacterium, or a production method therefor.

The *Ruminococcaceae* enterobacterium used in the pharmaceutical composition of the present invention is preferably one having 16S rRNA gene with 95% or higher, 96% or higher, 97% or higher, 98% or higher, or 99% or higher sequence identity to the nucleotide sequence set forth in SEQ ID NO: 1. As such a bacterium, a bacterium isolated by the above isolation method may be suitably used.

Examples of the *Ruminococcaceae* enterobacterium used in a pharmaceutical composition of the present invention include *Ruminococcaceae* YB328 isolated by the present inventors. *Ruminococcaceae* YB328 has 16S rRNA gene with the nucleotide sequence set forth in SEQ ID NO: 1. The present applicant, by itself, has deposited *Ruminococcaceae* YB328, which is maintained and preserved in RIKEN. The present applicant guarantees that *Ruminococcaceae* YB328 can be transferred to a third party in compliance with the respective laws and regulations if any of the items of Article 27-3 of the Ordinance for Enforcement of the Japanese Patent Law is applicable.

*Ruminococcaceae* YB328, for example, can be suitably cultured at 37°C in an anaerobic chamber while using, for instance, the above EG medium.

The pharmaceutical composition of the present invention may contain multiple species of *Ruminococcaceae* enterobacterium or a single species of *Ruminococcaceae* enterobacterium. Examples of the *Ruminococcaceae* enterobacterium, the single species of which can exert the effect, can include *Ruminococcaceae* YB328. A composition containing an extremely wide variety of bacteria, such as the intestinal contents themselves, may be transplanted into a human body. This may cause a risk of adverse reactions such as infections and/or allergic reactions. The present invention, however, uses multiple species of *Ruminococcaceae* enterobacterium or a single species of *Ruminococcaceae* enterobacterium. This makes it possible to avoid such a risk.

The present invention provides a method for producing a pharmaceutical composition, the method comprising the step of blending the above *Ruminococcaceae* enterobacterium. The method for producing a pharmaceutical composition according to the present invention may include the step of blending a *Ruminococcaceae* enterobacterium isolated by the above method of isolating a *Ruminococcaceae* enterobacterium.

<Cells, culture supernatant, metabolite, and/or cell extract of *Ruminococcaceae* enterobacterium>

The pharmaceutical composition of the present invention comprises bacterial cells, a culture supernatant, a metabolite, and/or a bacterial cell extract of *Ruminococcaceae* enterobacterium. In the case of using cells, viable cells are preferred. The viable cells may be in the form of a culture containing a medium for culturing a *Ruminococcaceae* enterobacterium or in the form of lyophilized cells. The culture supernatant of *Ruminococcaceae* enterobacterium used in the present invention is a liquid portion of the bacterial culture after the bacteria have been removed by centrifugation or other methods. The metabolite of *Ruminococcaceae* enterobacterium used in the present invention may be purified, if appropriate, from the above culture, culture supernatant or the like. The cell extract of *Ruminococcaceae* enterobacterium used in the present invention refers to an extract obtained by breaking down the cells by a process such as crushing, sonication, dissolution by alkaline treatment etc. and suitably fractionating and/or purifying the extract as desired. It is possible to use, if appropriate, for example, cell contents, cell membrane components, their purified products, or a combination thereof.

<Immune checkpoint inhibitor>

The pharmaceutical composition of the present invention may be administered in combination with an immune checkpoint inhibitor. As used herein, the immune checkpoint inhibitor means an agent that has a function to inhibit the function of an immune checkpoint molecule and to release the suppression of T-cell responses. The immune checkpoint inhibitor used in the present invention is preferably a human immune checkpoint inhibitor. For example, it is suitable to be able to use an inhibitor for any of immune checkpoint molecules selected from the group consisting of PD-1, CTLA-4, TIM-3, BTLA, LAG-3, A2aR, KIR, VISTA, TIGIT, PD-L1 PD-L2, CD80, CD86, GAL-9, HVEM, CD160, MHC class II, B7-H3, B7-H4, B7-H5, B7-H6, and B7-H7, or a combination of two or more different inhibitors therefor. The immune checkpoint inhibitor used in the present invention is suitably an antibody against the immune checkpoint molecule, an antigen-binding fragment of the antibody, or a combination thereof. For example, the immune checkpoint inhibitor is selected from the group consisting of nivolumab, pembrolizumab, cemiplimab, avelumab, atezolizumab, and durvalumab.

<Pharmaceutical composition>

The form of the pharmaceutical composition of the present invention is not particularly limited, but depending on the purpose, any of tablets, powders, granules, capsules, enteric capsules, suppositories, liquids, suspensions, gels, or other forms may be selected, if appropriate. The pharmaceutical composition of the present invention comprises bacterial cells, a culture supernatant, a metabolite, and/or a bacterial cell extract of *Ruminococcaceae* enterobacterium. It is possible to use a composition formulated using, in addition to the bacterial cells, the culture supernatant, the metabolite, and/or the bacterial cell extract of *Ruminococcaceae* enterobacterium, a pharmaceutically acceptable carrier, diluent, and/or filler.

The method of administering a pharmaceutical composition according to the present invention is not particularly limited, and may be set, if appropriate, in consideration of the form of preparation, the age and sex of each patient, the degree of disease, and so on. For example, an administration method such as oral, tubal or enema administration may be suitably used.

The pharmaceutical composition of the present invention may be administered in combination with an immune checkpoint inhibitor. Specifically, the pharmaceutical composition containing bacterial cells, a culture supernatant, a metabolite, and/or a bacterial cell extract of *Ruminococcaceae* enterobacterium may be administered simultaneously with or separately from administration of an immune checkpoint inhibitor. The pharmaceutical composition may also be administered before or after administration of an immune checkpoint inhibitor. The pharmaceutical composition used in the present invention may be in the form of a combination prepared by blending an immune checkpoint inhibitor and bacterial cells, a culture supernatant, a metabolite, and/or a bacterial cell extract of *Ruminococcaceae* enterobacterium in a single preparation.

The dosage regimen of a pharmaceutical composition of the present invention may be set, if appropriate, in consideration of the form of preparation, the age and sex of each patient, the degree of disease, and so on. The *Ruminococcaceae* enterobacterium is usually daily administered preferably at about $1 \times 10^8$ to $1 \times 10^{11}$ cells per patient, and more preferably at about $1 \times 10^9$ to $1 \times 10^{10}$ cells per patient.

Another aspect of the present invention provides a pharmaceutical composition comprising bacterial cells, a culture supernatant, a metabolite, and/or a bacterial cell extract of *Ruminococcaceae* enterobacterium for activating CD8-positive T cells in a subject.

As described below, the *Ruminococcaceae* enterobacterium in the present invention has a better effect of activating CD8-positive T cells ($CD8^+$ T cells) through maturation of dendritic cells than the case where other bacteria (e.g., *B. vulgatus)* or a vehicle (e.g., PBS or saline) is administered. The activation of $CD8^+$ T cells may be analyzed, for example, by measuring the expression level of IFN-γ protein or a polynucleotide encoding it. The expression level of protein or polynucleotide may be analyzed by suitably using a known technique such as quantitative protein expression analysis (e.g., flow cytometry, Western blotting) or quantitative gene expression analysis (e.g., transcriptome analysis, real-time quantitative PCR). Another aspect of the present invention provides a pharmaceutical composition comprising bacterial cells, a culture supernatant, a metabolite, and/or a bacterial cell extract of *Ruminococcaceae* enterobacterium, wherein the pharmaceutical composition is administered in combination with an immune checkpoint inhibitor, the pharmaceutical composition enhancing the immune response against tumor or cancer in a subject with a tumor or cancer.

<Tumor or cancer>

Examples of the tumor or cancer herein include, but are not limited to, malignant pleural mesothelioma, malignant peritoneal mesothelioma, malignant melanoma, malignant lymphoma, brain tumor, glioma, neuroblastoma, thymoma, gastrointestinal stromal tumor, neuroendocrine tumor, testicular tumor, soft tissue sarcoma, nephroblastoma, hepatoblastoma, germ cell tumor, retinoblastoma, osteosarcoma, Ewing sarcoma, rhabdomyosarcoma, acute myelogenous leukemia, acute lymphocytic leukemia, chronic myelogenous leukemia, chronic lymphocytic leukemia, myelodysplastic syndrome, adult T-cell leukemia, multiple myeloma, oropharyngeal cancer, laryngeal cancer, tongue cancer, nasal cancer, sinus cancer, thyroid cancer, parotid cancer, submandibular gland cancer, auditory cancer, lung cancer, breast cancer, thymic cancer, esophageal cancer, gastric cancer, colon cancer, small intestine cancer, hepatocellular carcinoma, bile duct cancer, gallbladder cancer, pancreatic cancer, renal cell carcinoma, renal pelvis and ureter cancer, bladder cancer, ureteral duct cancer, adrenal carcinoma, peritoneal carcinoma, prostate cancer, cervical cancer, uterine cancer, ovarian cancer, vaginal cancer, vulvar cancer, basal cell carcinoma, spinous cell carcinoma, neuroendocrine cancer, Kaposi sarcoma, and cancer of unknown primary origin.

<Effect of enhancing immune response>

The case where an immune checkpoint inhibitor is administered alone in a subject may be compared to the case where a pharmaceutical composition of the present invention and the immune checkpoint inhibitor are used in combination and administered. Then, an indicator for an activated immune response may be used to evaluate whether the immune response against tumor or cancer is enhanced. Examples of the indicator for an activated immune response include: but are not limited to, proliferation and/or activation of cytotoxic T cells or their progenitors $CD8^+$ T cells; an increased percentage of $CD62L^-CD44^+$ cells in $CD8^+$ T cells; an increased percentage of TNF-$\alpha^+$IFN-$\gamma^+$ cells in $CD8^+$ T cells; a decreased number of regulatory T cells (Treg, $CD4^+CD25^+FoxP3^+$ cells); an increased ratio of $CD4^+$ cell count with respect to $FoxP3^+$ cell count; increased expression of dendritic cell maturation markers (e.g., CD80, CD86, MHC class I); increased expression of activation markers (e.g., IFN-$\gamma$) on $CD8^+$ T cells; increased expression of TCR signaling (e.g., ZAP70); or increased expression of CD28 signaling (e.g., Erk (pErk), Akt (pAkt), S6 (pS6)).

Another aspect of the present invention provides a pharmaceutical composition comprising bacterial cells, a culture supernatant, a metabolite, and/or a bacterial cell extract of *Ruminococcaceae* enterobacterium, wherein the pharmaceutical composition is administered in combination with an immune checkpoint inhibitor, the pharmaceutical composition being used for treating a tumor or cancer in a subject.

<Effect of treating tumor or cancer>

The tumor or cancer may be eliminated, reduced in the size, or stabilized without any size change in the case where a pharmaceutical composition of the present invention and an immune checkpoint inhibitor are used in combination and administered when compared to the case where the immune checkpoint inhibitor is administered alone. The former case can be evaluated such that the administration of the pharmaceutical composition of the present invention in combination with the immune checkpoint inhibitor has exerted therapeutic effects on the tumor or cancer.

CR (Complete Response), PR (Partial Response), SD (Stable Disease), and PD (Progressive Disease) are common criteria used in the art as indicators for eliminating, reducing, or stabilizing a tumor or cancer, and may be adopted. CR is complete disappearance of tumor, PR is a reduction in the total tumor size by 30% or more, SD is a state without any change in tumor size, and PD is an increase in the total tumor size by 20% or more and an absolute value increase by 5 mm or more, or appearance of a new lesion. When herein evaluated as CR, PR, or SD, the therapeutic effect is present in the tumor or cancer.

Another aspect of the present invention provides a pharmaceutical composition comprising bacterial cells, a culture supernatant, a metabolite, and/or a bacterial cell extract of *Ruminococcaceae* enterobacterium, wherein the pharmaceutical composition is administered in combination with an immune checkpoint inhibitor, the pharmaceutical composition being used for suppressing recurrence or metastasis of a tumor or cancer in a subject.

<Effect of suppressing recurrence or metastasis of tumor or cancer>

As used herein, the wording "recurrence of a tumor or cancer" means reappearance of a tumor or cancer in the vicinity of the treated tumor or cancer within 1 month, 6 months, 1 year, 3 years, 5 years, or 10 years after treatment of the tumor or cancer. In addition, as used herein, the wording "metastasis of a tumor or cancer" means occurrence of a tumor or cancer in a site distant from the treated tumor or cancer within 1 month, 6 months, 1 year, 3 years, 5 years, or 10 years after treatment of the tumor or cancer.

The case where an immune checkpoint inhibitor is administered alone may be compared to the case where a pharmaceutical composition of the present invention and the immune checkpoint inhibitor are used in combination and administered. In the latter case, no recurrence or metastasis of a tumor or cancer may be observed within 1 month, 6 months, 1 year, 3 years, 5 years, or 10 years after treatment for the tumor or cancer; or the timing of occurrence may be delayed, or the number of occurrences may be reduced. This case can be then evaluated such that the administration of the pharmaceutical composition of the present invention in combination with the immune checkpoint inhibitor has exerted an effect of suppressing recurrence or metastasis of a tumor or cancer.

The pharmaceutical composition of the present invention may be further administered in combination with at least one therapy selected from the group consisting of surgery, chemotherapy, and radiation therapy.

<Surgery, chemotherapy, and radiation therapy>

Examples of the surgery that can be used in combination with a pharmaceutical composition of the present invention include, but are not limited to, direct surgery or specular surgery for the purpose of, for instance, resection of a tumor or cancer lesion, resection of a tumor or cancer organ, dissection of lymph nodes near a tumor or cancer. The chemotherapy that can be used in combination with a pharmaceutical composition of the present invention refers to treatment with a drug for preventing the growth or proliferation of tumor or cancer cells or promoting their death. Examples include, but are not limited to, hormone therapy or molecular targeted therapy. The radiation therapy that can be used in combination with a pharmaceutical composition of the present invention refers to radiation treatment for the purpose of killing tumor or cancer cells, reducing a tumor or cancer, preventing recurrence or metastasis of a tumor or cancer, and relieving a symptom of a tumor or cancer. Examples include, but are not limited to, external or internal irradiation.

<Method of enhancing immune response, method of treating tumor or cancer, or method of suppressing recurrence or metastasis of tumor or cancer>

Another aspect of the present invention provides a method of enhancing the immune response against tumor or cancer, the method comprising administering to a subject with a tumor or cancer an effective amount of an immune checkpoint inhibitor in combination with an effective amount of a pharmaceutical composition containing bacterial cells, a culture supernatant, a metabolite, and/or a bacterial cell extract of *Ruminococcaceae* enterobacterium.

Another aspect of the present invention provides a method of treating a tumor or cancer, the method comprising administering to a subject with the tumor or cancer an effective amount of an immune checkpoint inhibitor in combination with an effective amount of a pharmaceutical composition containing bacterial cells, a culture supernatant, a metabolite, and/or a bacterial cell extract of *Ruminococcaceae* enterobacterium.

Another aspect of the present invention provides a method of suppressing recurrence or metastasis of a tumor or cancer in a subject, the method comprising administering to the subject with the tumor or cancer an effective amount of an immune checkpoint inhibitor in combination with an effective amount of a pharmaceutical composition containing bacterial cells, a culture supernatant, a metabolite, and/or a bacterial cell extract of *Ruminococcaceae* enterobacterium.

Each method described above may be further performed in combination with at least one therapy selected from the group consisting of surgery, chemotherapy, and radiation therapy.

<Pharmaceutical composition or method for increasing diversity of intestinal indigenous bacteria>

The bacterial cells, the culture supernatant, the metabolite, and/or the bacterial cell extract of *Ruminococcaceae* enterobacterium may be administered to a mammal. This can increase diversity of intestinal indigenous bacteria in the mammal when compared to that before the administration. Thus, a certain aspect of the present invention pertains to a pharmaceutical composition comprising bacterial cells, a culture supernatant, a metabolite, and/or a bacterial cell extract of *Ruminococcaceae* enterobacterium, the pharmaceutical composition being used to increase diversity of intestinal indigenous bacteria in a mammal when compared to that before administration. Another aspect of the present invention also pertains to a method of increasing the number of intestinal indigenous bacteria in a mammal when compared to that before administration, comprising administering to the mammal an effective amount of a pharmaceutical composition containing bacterial cells, a culture supernatant, a metabolite, and/or a bacterial cell extract of *Ruminococcaceae* enterobacterium.

For example, the Shannon-Wiener index (hereinafter, sometimes referred to as the Shannon index) may be used to measure the diversity of intestinal indigenous bacteria. The Shannon index is expressed by the following equation:

[Formula 1]

$$H' = -\sum_{i=1}^{S} p_i \ln p_i$$

where S is the number of species, pi is the percentage of the number of individuals of the i-th species (ni) with respect to the total number of individuals N, and $p_i$ = ndN.

The pharmaceutical composition containing bacterial cells, a culture supernatant, a metabolite, and/or a bacterial cell extract of *Ruminococcaceae* enterobacterium in the present invention may be administered to a mammal. By doing so, the Shannon index H' in the intestinal indigenous bacteria after administration can be increased, preferably significantly, compared to the Shannon index H' in the intestinal indigenous bacteria before administration. It is known that the diversity of intestinal indigenous bacteria is low in non-responders to an immune checkpoint inhibitor. The *Ruminococcaceae* enterobacterium in the present invention is highly effective in increasing the diversity of intestinal indigenous bacteria when compared to other bacteria. Although not bound by any particular theory, one mechanism, by which the *Ruminococcaceae* enterobacterium of the present invention can elicit an anti-tumor immune response, may be due to increased diversity of intestinal indigenous bacteria.

The pharmaceutical composition of the present invention for increasing diversity of intestinal indigenous bacteria in a mammal may be administered in combination with an immune checkpoint inhibitor.

<Pharmaceutical composition and method for inducing dendritic cell progenitors into type 1 dendritic cells>

As described below, the present inventors have found that a *Ruminococcaceae* enterobacterium can be used to induce dendritic cell progenitors into type 1 dendritic cells. Further, it has been found that simultaneous stimulation of multiple TLRs other than TLR4 is important for the induction of type 1 dendritic cells from dendritic cell progenitors.

Thus, the present invention also pertains to a pharmaceutical composition for inducing dendritic cell progenitors to type 1 dendritic cells, comprising an agonist(s) for multiple TLRs other than TLR4.

The dendritic cell progenitors refer to cells where the expression of dendritic cell maturation markers is lower than that of mature dendritic cells. Examples of each dendritic cell maturation marker include, but are not limited to, CD80, CD86, or MHC class I. The dendritic cell progenitors in the present invention are preferably bone marrow-derived dendritic cell progenitors. The dendritic cell progenitors differentiate in response to various stimuli and eventually differentiate into type 1 dendritic cells (also called standard type 1 dendritic cells, cDC1), type 2 dendritic cells (also called standard type 2 dendritic cells, cDC2), or plasmacytoid dendritic cells (pDC). As used herein, the term "type 1 dendritic cells" refers to CD103-positive CD11b-negative dendritic cells.

A pharmaceutical composition for inducing dendritic cell progenitors to type 1 dendritic cells according to the present invention comprises an agonist(s) for multiple TLRs other than TLR4. Examples of the multiple TLRs other than TLR4 can include, but are not limited to, multiple TLRs selected from the group consisting of TLR1, TLR2, TLR3, TLR5, TLR6, TLR7, TLR8, and TLR9. Examples of a TLR1/TLR2 agonist include Pam3CSK4. Examples of a TLR2 agonist include a histone. Examples of a TLR2/TLR6 agonist include zymosan or MALP-2. Examples of a TLR3 agonist include Poly(I)/Poly(C). Examples of a TLR4 agonist include LPS. Examples of a TLR5 agonist include flagellin. Examples of a TLR7 agonist include R837 (imiquimod). Examples of a TLR7/8 agonist include R848 (resiquimod). Examples of a TLR9 agonist include CpG oligodeoxynucleotide (CpG-ODN) such as ODN1826. These examples are not limited to them. The bacterial cells, the culture supernatant, the metabolite, and/or the bacterial cell extract of *Ruminococcaceae* enterobacterium may be used as an agonist included in the pharmaceutical composition for inducing dendritic cell progenitors into type 1 dendritic cells in the present invention.

The pharmaceutical composition for inducing dendritic cell progenitors to type 1 dendritic cells in the present invention may be used to treat a tumor or cancer in a subject. The pharmaceutical composition for inducing dendritic cell progenitors to type 1 dendritic cells in the present invention may be used to enhance the immune response against tumor or cancer in a subject with a tumor or cancer.

The present invention also pertains to a method of inducing dendritic cell progenitors to type 1 dendritic cells, comprising bringing an agonist(s) for multiple TLRs other than TLR4 in contact with the dendritic cell progenitors.

The method of inducing dendritic cell progenitors to type 1 dendritic cells in the present invention may be performed *in vivo* or *in vitro.*

The origin of the dendritic cell progenitors used in the method of inducing dendritic cell progenitors to type 1 dendritic cells in the present invention is not limited, but is preferably a human origin.

The present invention further pertains to type 1 dendritic cells induced by the above method of inducing dendritic cell progenitors into type 1 dendritic cells.

The type 1 dendritic cells induced by the method of inducing dendritic cell progenitors to type 1 dendritic cells in the present invention may be used to treat a tumor or cancer in a subject. The type 1 dendritic cells induced by the method of inducing dendritic cell progenitors to type 1 dendritic cells in the present invention may be used to enhance the immune response against tumor or cancer in a subject with a tumor or cancer.

Thus, the present invention also pertains to a pharmaceutical composition for treating a tumor or cancer in a subject, comprising type 1 dendritic cells induced by the method of inducing dendritic cell progenitors to type 1 dendritic cells. In addition, the present invention also pertains to a pharmaceutical composition for enhancing the immune response against tumor or cancer, comprising type 1 dendritic cells induced by the method of inducing dendritic cell progenitors to type 1 dendritic cells.

Hereinbelow, the present invention will be described with reference to Examples. However, the present invention is not limited to them. Those skilled in the art may make various changes and/or modifications to the present invention. They are also included in the scope of the present invention.

## EXAMPLES

<Metagenome analysis of intestinal indigenous bacteria in immune checkpoint inhibitor responders and non-responders>

By using intestinal content samples from human patients (43 gastric cancer patients and 18 lung cancer patients) treated with an immune checkpoint inhibitor nivolumab or pembrolizumab, metagenome analysis was conducted on intestinal indigenous bacteria in responders and non-responders to the immune checkpoint inhibitor. Patients who achieved a partial response (PR) or better or stable disease (SD) for 6 months or longer by RECIST ver1.1 using CT imaging were defined as responders, and non-responders were defined as other cases.

Fig. 1 shows the results of LEfSe analysis in which intestinal indigenous bacteria were compared between the responders and the non-responders. A *Ruminococcaceae* enterobacterium group was identified as a frequently observed bacterial group in the responders, and a genus *Ruminococcus* bacterial group and a *Ruminococcus* unclassified genus bacterial group, which belong to the family *Ruminococcaceae,* were also identified.

Next, the correlation was investigated between the difference in the percentage of each bacterial group in the intestinal indigenous bacteria and the progression free survival (PFS) of the patients. Specifically, the progression-free survival was compared between a group of patients with a high median percentage of *Ruminococcaceae* enterobacterium group in the intestinal indigenous bacteria (high *Ruminococcaceae* enterobacterium group) and a group of patients with a low median percentage of *Ruminococcaceae* enterobacterium group in the intestinal indigenous bacteria (low *Ruminococcaceae* enterobacterium group). The same applied to each of the *Ruminococcus* unclassified genus bacterial group, the genus *Ruminococcus* bacterial group, or a *Bacteroides* bacterial group. The progression-free survival was compared between the patients with a high median percentage of and the patients with a low median percentage of each bacterial group in the intestinal indigenous bacteria. Fig. 2 shows the results. The progression-free survival tended to be longer in the high *Ruminococcaceae* enterobacterium group, the high *Ruminococcus* unclassified genus bacterial group, and the high genus *Ruminococcus* bacterial group, whereas the progression-free survival tended to be shorter in the high *Bacteroides* bacterial group.

<Transplantation of intestinal contents from immune checkpoint inhibitor responders into mice>

The intestinal contents derived from the above responders or non-responders were suspended in an isotonic solution and prepared as a suspension. The suspension was administered to pathogen-free BALB/cAJcl mice to check how this affected efficacy of an immune checkpoint inhibitor (an anti-PD-1 antibody, Ultra-LEAF Purified anti-mouse CD279 (PD-1) (RMP1-14); purchased from BioLegend, Inc.). Mice were divided into four groups: immune checkpoint inhibitor-treated and non-treated groups in the responder intestinal content transplantation group and immune checkpoint inhibitor-treated and non-treated groups in the non-responder intestinal content transplantation group. An immune-responsive mouse tumor model was created by transplanting MC38 cultured cells subcutaneously in each mouse 14 days after administration of intestinal contents. Five, eight, and eleven days after transplantation of MC38 cultured cells, each treated group received an immune checkpoint inhibitor, while each non-treated group received PBS intraperitoneally. Subsequently, the tumor diameter and survival were observed and compared between each mouse. The tumor volume was calculated based on the measured tumor diameter by using the following calculation formula:

[Formula 2]

$$\text{Long diameter} \times \text{Short diameter} \times \text{Short diameter} \times \frac{1}{2}$$

Fig. 3 shows the results. The immune checkpoint inhibitor-treated group in the responder intestinal content transplantation group (the responder treated group) showed more marked tumor volume reduction and prolonged survival effects than any of the immune checkpoint inhibitor-nontreated group in the responder intestinal content transplantation group (the responder non-treated group), the immune checkpoint inhibitor-treated group in the non-responder intestinal content transplantation group (the non-responder treated group), and the immune checkpoint inhibitor-nontreated group in the non-responder intestinal content transplantation group (the non-responder non-treated group). In other words, the results indicate that the transplantation of intestinal contents from responders has the efficacy of enhancing the effect of an immune checkpoint inhibitor against tumor, i.e., the effect of enhancing an immune response against tumor. In addition, the responder non-treated group showed more tumor volume reduction and prolonged survival effects than the non-responder non-treated group. Therefore, the transplantation of responder intestinal contents elicited a synergistic anti-tumor effect when combined with immune checkpoint inhibitor therapy, while the transplantation of responder intestinal contents alone had an anti-tumor effect.

<Transplantation of intestinal contents derived from immune checkpoint inhibitor responders into antibiotic-treated mice>

The above intestinal content transplantation experiments were performed using SPF mice that had been previously treated with antibiotics.

The intestinal contents derived from the above responders or non-responders were suspended in an isotonic solution and prepared as a suspension. The suspension was administered to pathogen-free BALB/cAJcl mice, to which antibiotics (ampicillin, vancomycin, neomycin, and metronidazole) had been administered for 6 days, to check how this affected efficacy of an immune checkpoint inhibitor (an anti-PD-1 antibody, Ultra-LEAF Purified anti-mouse CD279 (PD-1) (RMP1-14); purchased from BioLegend, Inc.). The mice were divided into four groups: immune checkpoint inhibitor-treated (responder intestinal content transplantation+, anti-PD-1 antibody (anti-PD-1 mAb)+) and non-treated (responder intestinal content transplantation-)-, isotype control+) groups in the responder intestinal content transplantation group and immune checkpoint inhibitor-treated (non-responder intestinal content transplantation+, anti-PD-1 antibody+) and non-treated (non-responder intestinal content transplantation+, isotype control+) groups in the non-responder intestinal content transplantation group. An immune-responsive mouse tumor model was created by transplanting MC38 cultured cells subcutaneously in each mouse 14 days after administration of intestinal contents. Five, eight, and eleven days after transplantation of MC38 cultured cells, the immune checkpoint inhibitor or the isotype control antibody (Ultra-LEAF Purified Rat IgG2a, $\kappa$ isotype Ctrl (RTK2758); purchased from BioLegend, Inc.) was intraperitoneally administered to the treated-group or the non-treated group, respectively. Fourteen days after, the mice were euthanized, and lymphocytes were isolated from the recovered tumors and analyzed for tumor-infiltrating T cells by using flow cytometry. Fig. 4 shows the results. The results showed that $CD8^+$ cells in the responder treated group had a significantly greater percentage of $CD62L^-CD44^+$ cell fraction than those in any of the responder non-treated, non-responder treated, or non-responder non-treated group. The results further showed that $CD8^+$ cells in the responder treated group also had a significantly greater percentage of TNF-$\alpha^+$IFN-$\gamma^+$ cell fraction than those in any of the responder non-treated, non-responder treated, or non-responder non-treated group. In other words, the results showed that the responder treated group had a significantly greater percentage of effector cells in the $CD8^+$ cells and a larger amount of $CD8^+$ cell-produced cytokines than any of the responder non-treated, non-responder treated, or non-responder non-treated group. This indicates that the transplantation of intestinal contents even from responders alone exerted a remarkable immune response-enhancing effect and the transplantation elicits a synergistic immune response-enhancing effect when combined with an immune checkpoint inhibitor.

<Isolation of *Ruminococcaceae* enterobacterium from intestinal contents from immune checkpoint inhibitor responders>

According to the description in "The World of Enterobacteria" by Tomotari Mitsuoka (1990), Asakura Publishing Co., Ltd., the above responders-derived intestinal contents were diluted in anaerobic diluent (B) in the book to prepare a diluted intestinal content liquid. The diluted intestinal content liquid prepared by dilution at $10^{-7}$, $10^{-8}$, or $10^{-9}$ was each inoculated into an EG agar medium, and cultured in an anaerobic chamber at 37°C for 3 to 4 days to form colonies.

The 16S rRNA gene sequence was determined for each bacterial colony formed, and phylogenetic analysis was performed according to a conventional procedure. Fig. 5 shows the results. A bacterium having 16S rRNA gene with the nucleotide sequence set forth in SEQ ID NO: 1 was named *Ruminococcaceae* YB328.

<Transplantation of each bacterium species into immune checkpoint inhibitor-treated tumor mice>

MC38 cultured cells were transplanted subcutaneously in pathogen-free BALB/cAJcl mice treated with antibiotics (ampicillin, vancomycin, neomycin, and metronidazole) for 6 days, and an immune checkpoint inhibitor (anti-PD-1 antibody) was administered 5, 8, and 11 days later. The control group received an isotype control. For the single bacterium transplantation group, each bacterium (*Akkermansia muchiniphilliam, Eggerhella lenta, Clostridum colicanis, Bacteroides vulgatus (B. vulgatus), Ersipelatoctostridum ransam,* or *Ruminococcaceae* YB328) alone was administered orally while simultaneously administering an immune checkpoint inhibitor or an isotype control. The tumor diameter was then measured for each mouse, and a plot of the tumor volume calculated based on the measurements is shown in Fig. 6. The group treated with *Ruminococcaceae* YB328 showed more marked tumor volume reduction when compared to the group treated with an immune checkpoint inhibitor alone as well as when compared to the group transplanted with another bacterium species. In other words, the results showed that *Ruminococcaceae* YB328, a *Ruminococcaceae* enterobacterium derived from the intestinal contents of responders, showed a synergistic anti-tumor effect when combined with an immune checkpoint inhibitor.

Similar experiments were performed on *B. vulgatus* and *Ruminococcaceae* YB328. Fourteen days after MC38 cultured cell were subcutaneously transplanted, the mice were euthanized, and lymphocytes were isolated from the recovered tumors and analyzed for tumor-infiltrating T cells by using flow cytometry. Fig. 7 shows the results. The results showed that the *Ruminococcaceae* YB328-treated group had a significantly larger percentage of $CD62L^{-}CD44^{+}$ cell fraction in the $CD8^{+}$ cells than the *B. vulgatus-treated* group or the group treated with an immune checkpoint inhibitor alone. The results further showed that the *Ruminococcaceae* YB328-treated group had a significantly larger percentage of TNF-$\alpha$+IFN-$\gamma^{+}$ cell fraction in the $CD8^{+}$ cells than the *B. vulgatus-treated* group or the group treated with an immune checkpoint inhibitor alone. That is, the results showed that the *Ruminococcaceae* YB328-treated group had a significantly larger percentage of effector cells in the $CD8^{+}$ cells and a larger amount of $CD8^{+}$ cell-produced cytokines than the *B. vulgatus*-treated group or the group treated with an immune checkpoint inhibitor alone. This indicates that *Ruminococcaceae* YB328 exerts a synergistic immune response-enhancing effect when combined with an immune checkpoint inhibitor.

<Influence of *Ruminococcaceae* enterobacterium on T cell activation>

Mouse bone marrow-derived dendritic cells (synonymous with dendritic cell progenitors) were co-cultured with *Ruminococcaceae* YB328 or *B. vulgatus* or vehicle. Then, dendritic cell maturation markers (CD80, CD86, MHC class I) were measured using flow cytometry. Fig. 8 shows the results. All the measured dendritic cell maturation markers in the case of co-culture with *Ruminococcaceae* YB328 were found to have a significantly increased level of expression when compared to the case of co-culture with B. *vulgatus* or vehicle. In other words, *Ruminococcaceae* YB328 was demonstrated to have significantly higher dendritic cell maturation effects than *B. vulgatus* or vehicle.

Next, dendritic cells after the above co-culture were collected, co-cultured with $CD8^{+}$ T cells derived from OT-I mice, and stimulated with N4 peptide (at 1 nM, 10 nM, or 100 nM) or Q4H7 peptide (at 1 nM, 10 nM or 100 nM), which peptides are known OVA antigen peptides with different affinities for TCR. Note that the Q4H7 peptide has been found to have lower affinity for TCR than the N4 peptide. Subsequently, a $CD8^{+}$ T cell activation marker (IFN-$\gamma$) was measured using ELISA, and TCR signaling (ZAP70 (pZAP70)) and CD28 signaling (Erk (pErk), Akt (pAkt), S6 (pS6)) were measured using flow cytometry.

Fig. 9 shows the results of measuring the $CD8^{+}$ T cell activation marker (IFN-$\gamma$) when stimulated with 1 nM or 100 nM of N4 peptide. Dendritic cells co-cultured with *Ruminococcaceae* YB328 were co-cultured with $CD8^{+}$ T cells. This case exhibited a significantly higher IFN-$\gamma$ production in response to the N4 peptide stimulation than the case where dendritic cells co-cultured with *B. vulgatus* were co-cultured with $CD8^{+}$ T cells. That is, the dendritic cells co-cultured with *Ruminococcaceae* YB328 were demonstrated to exert a significantly higher $CD8^{+}$ T cell activation effect than the dendritic cells co-cultured with B. *vulgatus.* Surprisingly, this effect was also demonstrated by N4 peptide at a concentration as low as 1 nM. This has suggested a high immune response-enhancing effect of *Ruminococcaceae* YB328.

Fig. 10 shows the results of measuring TCR signaling (ZAP70) and CD28 signaling (Erk) when stimulated with N4 peptide or Q4H7 peptide. The case where dendritic cells co-cultured with *Ruminococcaceae* YB328 were co-cultured with $CD8^{+}$ T cells was found to generate significantly higher TCR signaling (ZAP70) and CD28 signaling (Erk) in response to N4 peptide stimulation or Q4H7 peptide stimulation than the case where dendritic cells co-cultured with *B. vulgatus* were co-cultured with $CD8^{+}$ T cells or the case where normal dendritic cells were co-cultured with $CD8^{+}$ T cells. That is, the dendritic cells co-cultured with *Ruminococcaceae* YB328 were demonstrated to exert a significantly higher effect of activating both the TCR signaling and the CD28 signaling than the dendritic cells co-cultured with *B. vulgatus.* Surprisingly, this effect was also demonstrated by the Q4H7 peptide with low TCR affinity, which peptide normally seems to contribute little to T cell activation.

Fig. 11 shows the results of investigating how stimulation of N4 peptide with different concentrations (0 nM, 1 nM, 10 nM, or 100 nM) affected TCR signaling (ZAP70 (pZAP70)) and CD28 signaling (Erk (pErk), Akt (pAkt), S6 (pS6)) when dendritic cells co-cultured with *Ruminococcaceae* YB328 were co-cultured with $CD8^{+}$ T cells or when dendritic cells co-cultured with *B. vulgatus* were co-cultured with $CD8^{+}$ T cells, respectively. The case where dendritic cells co-cultured with *Ruminococcaceae* YB328 were co-cultured with $CD8^{+}$ T cells was found to generate a significantly higher level of each signaling in response to stimulation of N4 peptide at every concentration examined than the case where dendritic cells co-cultured with *B. vulgatus* were co-cultured with $CD8^{+}$ T cells. That is, the dendritic cells co-cultured with *Ruminococcaceae* YB328 were demonstrated to exert a significantly higher effect of activating both the TCR signaling and the CD28 signaling by stimulation of N4 peptide at a low concentration than the dendritic cells co-cultured with B. *vulgatus.*

The above results indicate that *Ruminococcaceae* YB328 can induce maturation of dendritic cells, which are antigen-presenting cells, and thereby exerts a larger effect of activating two pathways (TCR and CD28) required for T cell activation even in the case of using low-concentration or low-affinity antigen stimulation. This suggests that each *Ruminococcaceae* enterobacterium such as *Ruminococcaceae* YB328 elicits a high immune response-enhancing effect *in vivo.*

<Effect of *Ruminococcaceae* enterobacterium in intestinal contents-transplanted tumor-bearing mice>

Pathogen-free BALB/cAJcl mice treated with antibiotics (ampicillin, vancomycin, neomycin, and metronidazole) for 6 days were transplanted with intestinal indigenous bacteria by administering a suspension of intestinal contents derived from the above-mentioned immune checkpoint inhibitor responders or non-responders. Then, MC38 cultured cells were subcutaneously transplanted into the mice, and 5, 8, and 11 days later, an immune checkpoint inhibitor (an anti-PD-1 antibody, Ultra-LEAF Purified anti-mouse CD279 (PD-1) (RMP1-14); purchased from BioLegend, Inc.) was intraperitoneally administered to the mice. In addition, *Ruminococcaceae* YB328 bacterium alone or *B. vulgatus* bacterium alone was administered orally. The tumor diameter was then measured for each mouse, and a plot of the tumor volume calculated based on the measurements is shown in Fig. 12. Here, Fig. 13 shows the results of conducting meta-analysis based on 16S rRNA gene in intestinal contents of the mice as collected either after transplantation of intestinal contents or after administration of single bacterium alone, and comparing the diversity of bacterial flora in the intestinal contents in each case.

Fig. 12 shows that tumor growth was significantly suppressed, in the *Ruminococcaceae* YB328 single bacterium administration group, for any of mice transplanted with either the intestinal contents derived from the responders or those from the non-responders. On the other hand, in the *B. vulgatus* single bacterium administration group, tumor growth could not be suppressed in any of mice transplanted with either the intestinal contents derived from the responders or those from the non-responders.

Fig. 13 has demonstrated that the diversity of bacterial flora was significantly increased in the intestinal contents after administration of the *Ruminococcaceae* YB328 bacterium alone when compared to that before the administration. It is known that the diversity of intestinal indigenous bacteria is low in non-responders to an immune checkpoint inhibitor. This experiment has revealed that the *Ruminococcaceae* enterobacterium in the present invention is highly effective in increasing the diversity of intestinal indigenous bacteria when compared to other bacteria. Although not bound by any particular theory, one mechanism, by which the *Ruminococcaceae* enterobacterium of the present invention can elicit an anti-tumor immune response, may be caused by increased diversity of intestinal indigenous bacteria.

<Effects of *Ruminococcaceae* enterobacterium on dendritic cell differentiation *(in vitro)*>

Transcriptome analysis was performed after mouse bone marrow-derived dendritic cells were co-cultured with *Ruminococcaceae* YB328 or *B. vulgatus* or LPS or vehicle (PBS) and RNA was then extracted from the corresponding dendritic cells. The results have indicated that dendritic cells stimulated with *Ruminococcaceae* YB328 exhibited higher expression of genes characteristic of type 1 dendritic cells (cDC1), such as batf3, Irf8, and FLT3, when compared to dendritic cells stimulated with *B. vulgatus* or LPS or vehicle (PBS) (Fig. 14).

<Effects of *Ruminococcaceae* enterobacterium on dendritic cell differentiation *(in vivo):* Part 1>

MC38 cultured cells were subcutaneously transplanted into pathogen-free BALB/cAJcl mice treated with antibiotics (ampicillin, vancomycin, neomycin, and metronidazole) for 6 days, and 5 days later, *Ruminococcaceae* YB328 or *B. vulgatus* was administered orally. After 8 days, each tissue (lymph node near the tumor, mucosa lamina propria, and intestinal peritoneal lymph node) was collected.

The results of FACS analysis showed that the *Ruminococcaceae* YB328 administration group had a significantly higher percentage of CD103-positive migratory dendritic cells in the lymph node near the tumor and CD103-positive CD11b-negative dendritic cells in the mucosal lamina propria. In addition, the *Ruminococcaceae* YB328 administration group was found to have an increased level of CCR7 expression in the mesenteric lymph node (Fig. 15). This has suggested that YB328 administration induces dendritic cells with a high migration potential and a high percentage of cDC1 *in vivo.*

<Effects of *Ruminococcaceae* enterobacterium on dendritic cell differentiation *(in vivo):* Part 2>

MC38 cultured cells were transplanted subcutaneously in pathogen-free BALB/cAJcl mice treated with antibiotics (ampicillin, vancomycin, neomycin, and metronidazole) for 6 days. An immune checkpoint inhibitor (anti-PD-1 antibody, RMP1-14, BioLegend, Inc., USA) or an isotype control antibody (RTK2758, BioLegend, inc., USA) was administered by intravenous injection twice with a 3-day interval. *Ruminococcaceae* YB328 or *B. vulgatus* or LPS or vehicle (PBS) was administered orally 5, 8, and 11 days after subcutaneous transplantation of MC38 cultured cells, and tumors were collected 13 days later.

The results of FACS analysis showed that the *Ruminococcaceae* TB328 administration group had a significantly higher percentage of CD103-positive dendritic cells in the tumors (Fig. 16).

<Mechanism by which *Ruminococcaceae* enterobacterium induces dendritic cell differentiation>

Dendritic cell progenitors (bone marrow-derived dendritic cells) were isolated from mouse bone marrow-derived cells, and co-cultured with a FLT3 ligand (FLT3L), which is required for differentiation into cDC1, and *Ruminococcaceae* YB328 or *B. vulgatus* or LPS or vehicle (PBS). Then, the expression of IRF8 was analyzed by FACS. When a high concentration (100 ng/ml) of FLT3L was administered, each case was found to have a high level of IRF8 expression. When a low concentration (1 ng/ml) of FLT3L was administered, the IRF8 expression was maintained only in the *Ruminococcaceae* YBS328 administration group (Fig. 17). This has suggested that *Ruminococcaceae* YB328 may induce differentiation into cDC1 in a FLT3L-independent manner.

Further, bone marrow-derived dendritic cells were co-cultured for 4 hours with *Ruminococcaceae* YB328 or *B. vulgatus* or LPS or vehicle (PBS). Then, the expression of p-S6 kinase (p-S6K) or p-STAT3 was analyzed by FACS. Only in the *Ruminococcaceae* YB328 administration group, the expression of both p-S6K and p-STAT3 molecules was found to be high (Fig. 18).

FLT3L is thought to be involved in dendritic cell differentiation through activation of the PI3K-mTOR pathway. Based on the above results, *Ruminococcaceae* YB328 is thought to be involved in the induction of differentiation into by activating the PI3K-mTOR pathway instead of using FLT3L.

<Induction of dendritic cell progenitors into type 1 dendritic cells by combined TLR stimulation >

As mentioned above, it has become clear that *Ruminococcaceae* YB328 participates in the expression of various molecules involved in dendritic cell differentiation. In order to elucidate the upstream pathway of the induction of differentiation into by *Ruminococcaceae* YB328, the present inventors focused on and investigated TLRs as follows.

First, in the transcriptome analysis shown in Fig. 14, the present inventors focused on TLRs to analyze. The results showed that dendritic cell progenitors stimulated with *Ruminococcaceae* YB328 had a higher level of expression for a variety of TLRs than dendritic cell progenitors stimulated with *B. vulgatus* (Fig. 19, A). Among them, the expression of TLR1, TLR3, TLR5, TLR7, and TLR9 was high.

In addition, bone marrow-derived dendritic cells collected from MyD88 knockout mice (MyD88-/-) were stimulated with *Ruminococcaceae* YB328, followed by FACS analysis. As a result, CD103-positive CD11b-negative dendritic cells were not induced, which was similar to the case of stimulation with vehicle (Fig. 19, B). This suggests that the differentiation into CD103-positive dendritic cells as induced by *Ruminococcaceae* YB328 depends on MyD88-TLR signaling and that TLR signaling plays an important role.

In the above transcriptome analysis, TLR5, 7, and 9 were particularly highly expressed in the *Ruminococcaceae* YB328 group. Thus, mouse bone marrow-derived dendritic cells (intact dendritic cell progenitors without bacterial stimulation or the like) were treated with a various mixture of each TLR5, 7, and/or 9 agonist (flagellin, R848 (resiquimod), and/or ODN-1826, respectively). In dendritic cells treated with all TLR5, 7, and 9 agonists, CD103-positive CD11b-negative dendritic cells, i.e., type 1 dendritic cells, were significantly induced when compared to those treated with LPS or control (Fig. 19, C). In this experiment, it has also been found that the addition of LPS, a TLR4 agonist, to the mixture of TLR5, 7, and 9 agonists significantly reduced the induction into CD103-positive CD11b-negative dendritic cells, i.e., type 1 dendritic cells (Fig. 19, C). This suggests that simultaneous stimulation of multiple TLRs other than TLR4 can induce type 1 dendritic cells from dendritic cell progenitors. Besides, it is suggested that *Ruminococcaceae* YB328 can stimulate multiple TLRs involved in the induction into these type 1 dendritic cells.

## Claims

**1.** A method of isolating a *Ruminococcaceae* enterobacterium, comprising the steps of:

(i) producing a diluted intestinal content liquid by serial dilution, using an anaerobic diluent, of intestinal contents obtained from a mammal that has received an immune checkpoint inhibitor and that has been evaluated as PR (partial response) or better or SD (stable disease) for six months or longer by CT imaging after administration;
(ii) inoculating a portion of the diluted intestinal content liquid into a solid medium for culturing under anaerobic conditions to form, on the solid medium, a colony(s) derived from a single clone of microorganisms contained in the diluted intestinal content liquid;
(iii) confirming whether or not a bacterium contained in the colony has 16S rRNA gene with 95% or higher sequence identity to a nucleotide sequence set forth in SEQ ID NO: 1; and
(iv) obtaining the bacterium confirmed to have the 16S rRNA gene with 95% or higher sequence identity to the nucleotide sequence set forth in SEQ ID NO: 1.

2. The isolation method according to claim 1, wherein the immune checkpoint inhibitor is an inhibitor for any of immune checkpoint molecules selected from the group consisting of PD-1, CTLA-4, TIM-3, BTLA, LAG-3, A2aR, KIR, VISTA, TIGIT, PD-L1 PD-L2, CD80, CD86, GAL-9, HVEM, CD160, MHC class II, B7-H3, B7-H4, B7-H5, B7-H6, and B7-H7, or a combination of two or more inhibitors therefor.

3. The isolation method according to claim 2, wherein the immune checkpoint inhibitor is selected from an antibody against the immune checkpoint molecule, an antigen-binding fragment of the antibody, or a combination thereof.

4. The isolation method according to claim 3, wherein the immune checkpoint inhibitor is selected from the group consisting of nivolumab, pembrolizumab, cemiplimab, avelumab, atezolizumab, and durvalumab.

5. The isolation method according to any one of claims 1 to 4, wherein the mammal is a human.

6. A method for producing a pharmaceutical composition, comprising the step of making a pharmaceutical composition by blending bacterial cells, a culture supernatant, a metabolite, and/or a bacterial cell extract of *Ruminococcaceae* enterobacterium isolated by the isolation method according to any one of claims 1 to 5.

7. The production method according to claim 6, wherein the pharmaceutical composition is a pharmaceutical composition administered in combination with an immune checkpoint inhibitor.

8. A pharmaceutical composition produced by the production method according to claim 6 or 7.

9. A pharmaceutical composition comprising bacterial cells, a culture supernatant, a metabolite, and/or a bacterial cell extract of *Ruminococcaceae* enterobacterium, wherein the composition is administered in combination with an immune checkpoint inhibitor.

10. The pharmaceutical composition according to claim 9, wherein the cells of *Ruminococcaceae* enterobacterium are viable cells.

11. The pharmaceutical composition according to claim 9 or 10, wherein the *Ruminococcaceae* enterobacterium is a bacterium having 16S rRNA gene with 95% or higher identity to a nucleotide sequence set forth in SEQ ID NO: 1.

12. The pharmaceutical composition according to any one of claims 8 to 11, wherein the immune checkpoint inhibitor is an inhibitor for any of immune checkpoint molecules selected from the group consisting of PD-1, CTLA-4, TIM-3, BTLA, LAG-3, A2aR, KIR, VISTA, TIGIT, PD-L1 PD-L2, CD80, CD86, GAL-9, HVEM, CD160, MHC class II, B7-H3, B7-H4, B7-H5, B7-H6, and B7-H7, or a combination of two or more different inhibitors therefor.

13. The pharmaceutical composition according to claim 12, wherein the immune checkpoint inhibitor is selected from an antibody against the immune checkpoint molecule, an antigen-binding fragment of the antibody, or a combination thereof.

14. The pharmaceutical composition according to any one of claims 8 to 13, which is administered by oral, tubal, or enema administration.

15. The pharmaceutical composition according to any one of claims 8 to 14, wherein the bacterial cells, the culture supernatant, the metabolite, and/or the bacterial cell extract of *Ruminococcaceae* enterobacterium is administered simultaneously with the immune checkpoint inhibitor.

16. The pharmaceutical composition according to claim 15, comprising the immune checkpoint inhibitor and the bacterial cells, the culture supernatant, the metabolite, and/or the bacterial cell extract of *Ruminococcaceae* enterobacterium.

17. The pharmaceutical composition according to any one of claims 8 to 14, wherein the bacterial cells, the culture supernatant, the metabolite, and/or the bacterial cell extract of *Ruminococcaceae* enterobacterium and the immune checkpoint inhibitor are administered separately.

18. The pharmaceutical composition according to claim 17, wherein before administration of the immune checkpoint inhibitor, the bacterial cells, the culture supernatant, the metabolite, and/or the bacterial cell extract of *Ruminococcaceae* enterobacterium is administered.

**19.** The pharmaceutical composition according to claim 17, wherein after administration of the immune checkpoint inhibitor, the bacterial cells, the culture supernatant, the metabolite, and/or the bacterial cell extract of *Ruminococcaceae* enterobacterium is administered.

**20.** The pharmaceutical composition according to any one of claims 8 to 19 for activating CD8 positive T cells in a subject.

**21.** The pharmaceutical composition according to any one of claims 8 to 19 for enhancing the immune response against tumor or cancer in a subject with a tumor or cancer.

**22.** The pharmaceutical composition according to claim 21, wherein the effect of enhancing the immune response against tumor or cancer is greater than when the immune checkpoint inhibitor is administered alone.

**23.** The pharmaceutical composition according to any one of claims 8 to 22 for treating a tumor or cancer in a subject.

**24.** The pharmaceutical composition according to claim 23, wherein the treatment is to eliminate, reduce, or stabilize the tumor or cancer.

**25.** The pharmaceutical composition according to claim 24, wherein the effect of eliminating, reducing, or stabilizing the tumor or cancer is greater than when the immune checkpoint inhibitor is administered alone.

**26.** The pharmaceutical composition according to any one of claims 8 to 22 for suppressing recurrence or metastasis of a tumor or cancer in a subject.

**27.** The pharmaceutical composition according to claim 26, wherein the effect of suppressing the recurrence or metastasis of the tumor or cancer is greater than when the immune checkpoint inhibitor is administered alone.

**28.** The pharmaceutical composition according to any one of claims 21 to 27, wherein the pharmaceutical composition is further administered in combination with at least one therapy selected from the group consisting of surgery, chemotherapy, and radiation therapy.

**29.** The pharmaceutical composition according to any one of claims 8 to 19 for increasing diversity of intestinal indigenous bacteria in a mammal when compared to that before administration.

**30.** A pharmaceutical composition for inducing dendritic cell progenitors to type 1 dendritic cells, comprising an agonist for multiple TLRs other than TLR4.

**31.** The pharmaceutical composition according to claim 30, wherein the multiple TLRs are TLR5, TLR7, and TLR9.

**32.** The pharmaceutical composition according to claim 31, wherein the agonist is a combination of flagellin, R848 (resiquimod), and ODN1826.

**33.** The pharmaceutical composition according to claim 30 or 31, wherein the agonist is bacterial cells, a culture supernatant, a metabolite, and/or a bacterial cell extract of *Ruminococcaceae* enterobacterium.

**34.** The pharmaceutical composition according to any one of claims 30 to 33 for treating a tumor or cancer in a subject.

**35.** A method of inducing dendritic cell progenitors to type 1 dendritic cells, comprising bringing an agonist for multiple TLRs other than TLR4 in contact with the dendritic cell progenitors.

**36.** Type 1 dendritic cells induced by the method according to claim 35.

**37.** A pharmaceutical composition comprising the type 1 dendritic cells according to claim 36 for treating a tumor or cancer in a subject.

Fig. 1

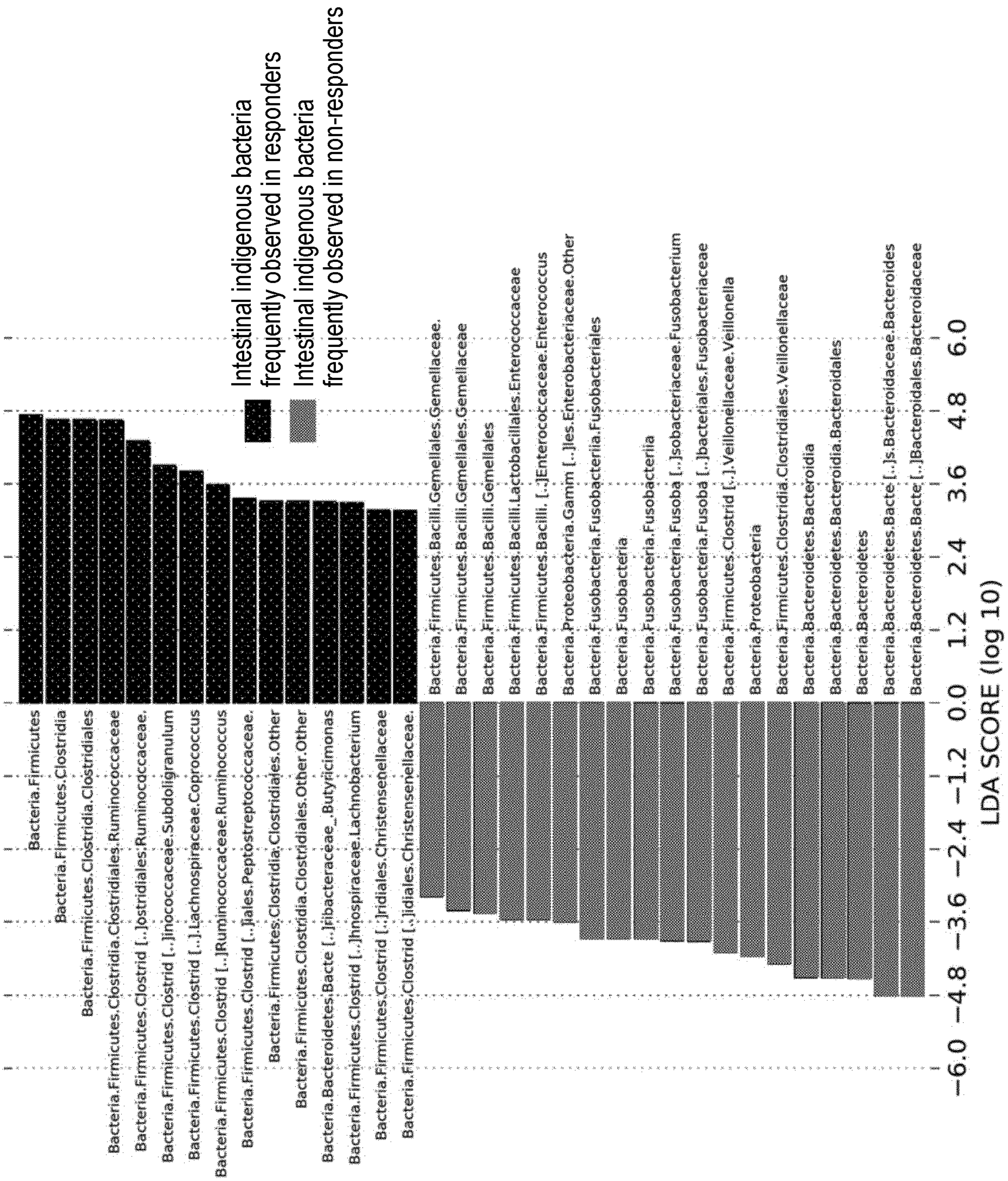
Intestinal indigenous bacteria frequently observed in responders
Intestinal indigenous bacteria frequently observed in non-responders
Bacteria.Firmicutes
Bacteria.Firmicutes.Clostridia
Bacteria.Firmicutes.Clostridia.Clostridiales
Bacteria.Firmicutes.Clostridia.Clostridiales.Ruminococcaceae
Bacteria.Firmicutes.Clostrid [..]ostridiales.Ruminococcaceae.
Bacteria.Firmicutes.Clostrid [..]inococcaceae.Subdoligranulum
Bacteria.Firmicutes.Clostrid [..].Lachnospiraceae.Coprococcus
Bacteria.Firmicutes.Clostrid [..]Ruminococcaceae.Ruminococcus
Bacteria.Firmicutes.Clostrid [..]iales.Peptostreptococcaceae.
Bacteria.Firmicutes.Clostridia.Clostridiales.Other
Bacteria.Firmicutes.Clostridia.Clostridiales.Other.Other
Bacteria.Bacteroidetes.Bacte [..]ribacteraceae._Butyricimonas
Bacteria.Firmicutes.Clostrid [..]hnospiraceae.Lachnobacterium
Bacteria.Firmicutes.Clostrid [..]ridiales.Christensenellaceae
Bacteria.Firmicutes.Clostrid [..]idiales.Christensenellaceae.
Bacteria.Firmicutes.Bacilli.Gemellales.Gemellaceae.
Bacteria.Firmicutes.Bacilli.Gemellales.Gemellaceae
Bacteria.Firmicutes.Bacilli.Gemellales
Bacteria.Firmicutes.Bacilli.Lactobacillales.Enterococcaceae
Bacteria.Firmicutes.Bacilli. [..]Enterococcaceae.Enterococcus
Bacteria.Proteobacteria.Gamm [..]les.Enterobacteriaceae.Other
Bacteria.Fusobacteria.Fusobacteria.Fusobacteriales
Bacteria.Fusobacteria
Bacteria.Fusobacteria.Fusobacteriia
Bacteria.Fusobacteria.Fusoba [..]sobacteriaceae.Fusobacterium
Bacteria.Fusobacteria.Fusoba [..]bacteriales.Fusobacteriaceae
Bacteria.Firmicutes.Clostrid [..].Veillonellaceae.Veillonella
Bacteria.Proteobacteria
Bacteria.Firmicutes.Clostridia.Clostridiales.Veillonellaceae
Bacteria.Bacteroidetes.Bacteroidia
Bacteria.Bacteroidetes.Bacteroidia.Bacteroidales
Bacteria.Bacteroidetes
Bacteria.Bacteroidetes.Bacte [..]s.Bacteroidaceae.Bacteroides
Bacteria.Bacteroidetes.Bacte [..]Bacteroidales.Bacteroidaceae
−6.0
−4.8
−3.6
−2.4
−1.2
0.0
1.2
2.4
3.6
4.8
6.0
LDA SCORE (log 10)

Fig. 2

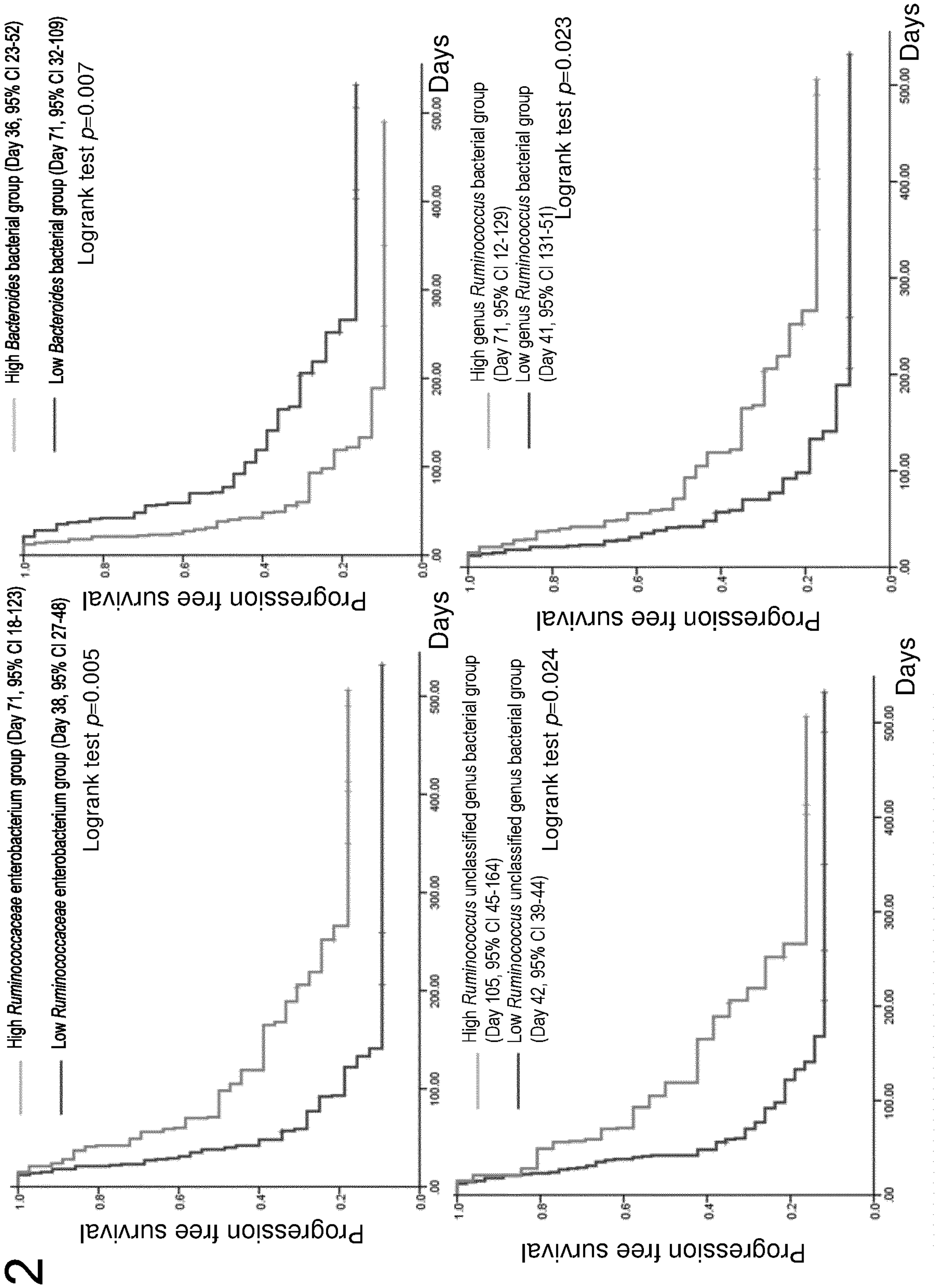
High *Ruminococcaceae* enterobacterium group (Day 71, 95% CI 18-123)
Low *Ruminococcaceae* enterobacterium group (Day 38, 95% CI 27-48)
Logrank test *p*=0.005
Progression free survival
Days
High *Bacteroides* bacterial group (Day 36, 95% CI 23-52)
Low *Bacteroides* bacterial group (Day 71, 95% CI 32-109)
Logrank test *p*=0.007
Progression free survival
Days
High *Ruminococcus* unclassified genus bacterial group
(Day 105, 95% CI 45-164)
Low *Ruminococcus* unclassified genus bacterial group
(Day 42, 95% CI 39-44)
Logrank test *p*=0.024
Progression free survival
Days
High genus *Ruminococcus* bacterial group
(Day 71, 95% CI 12-129)
Low genus *Ruminococcus* bacterial group
(Day 41, 95% CI 131-51)
Logrank test *p*=0.023
Progression free survival
Days

Fig. 3

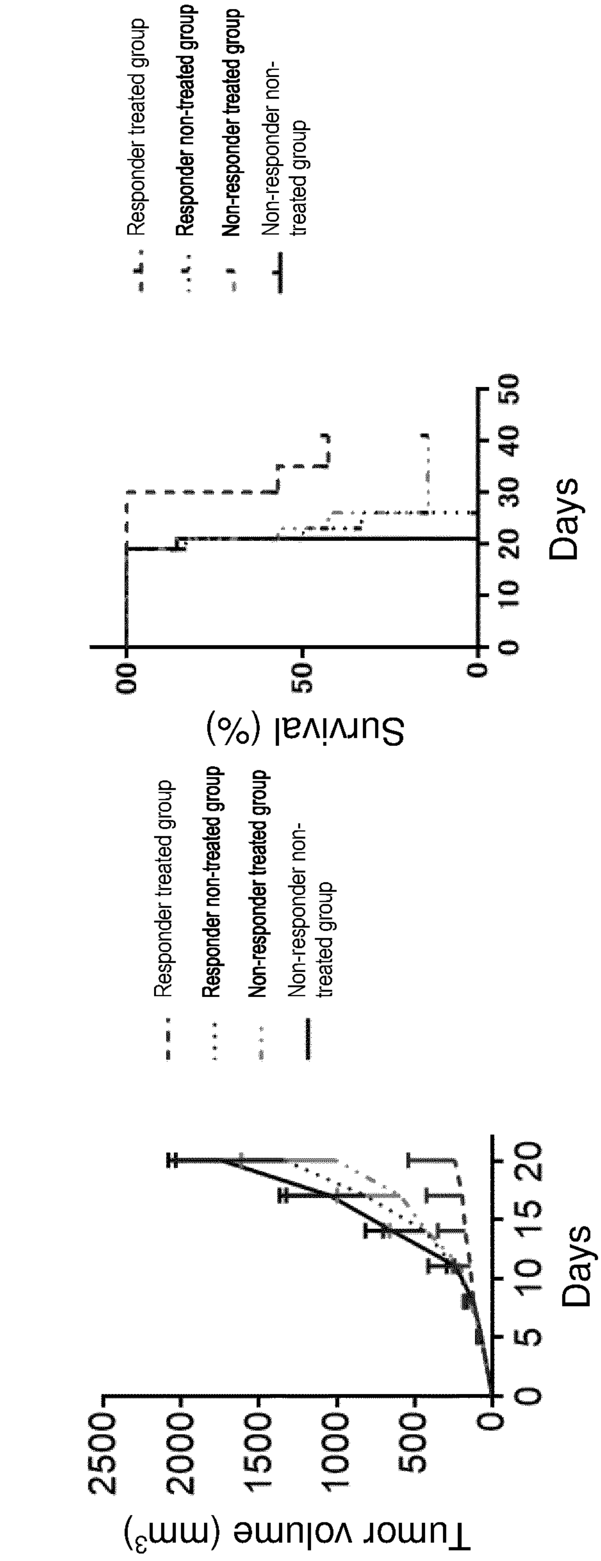
Tumor volume (mm3)
2500
2000
1500
1000
500
0
0
5
10
15
20
Days
Responder treated group
Responder non-treated group
Non-responder treated group
Non-responder non-treated group
Survival (%)
00
50
0
0
10
20
30
40
50
Days
Responder treated group
Responder non-treated group
Non-responder treated group
Non-responder non-treated group

Fig. 4

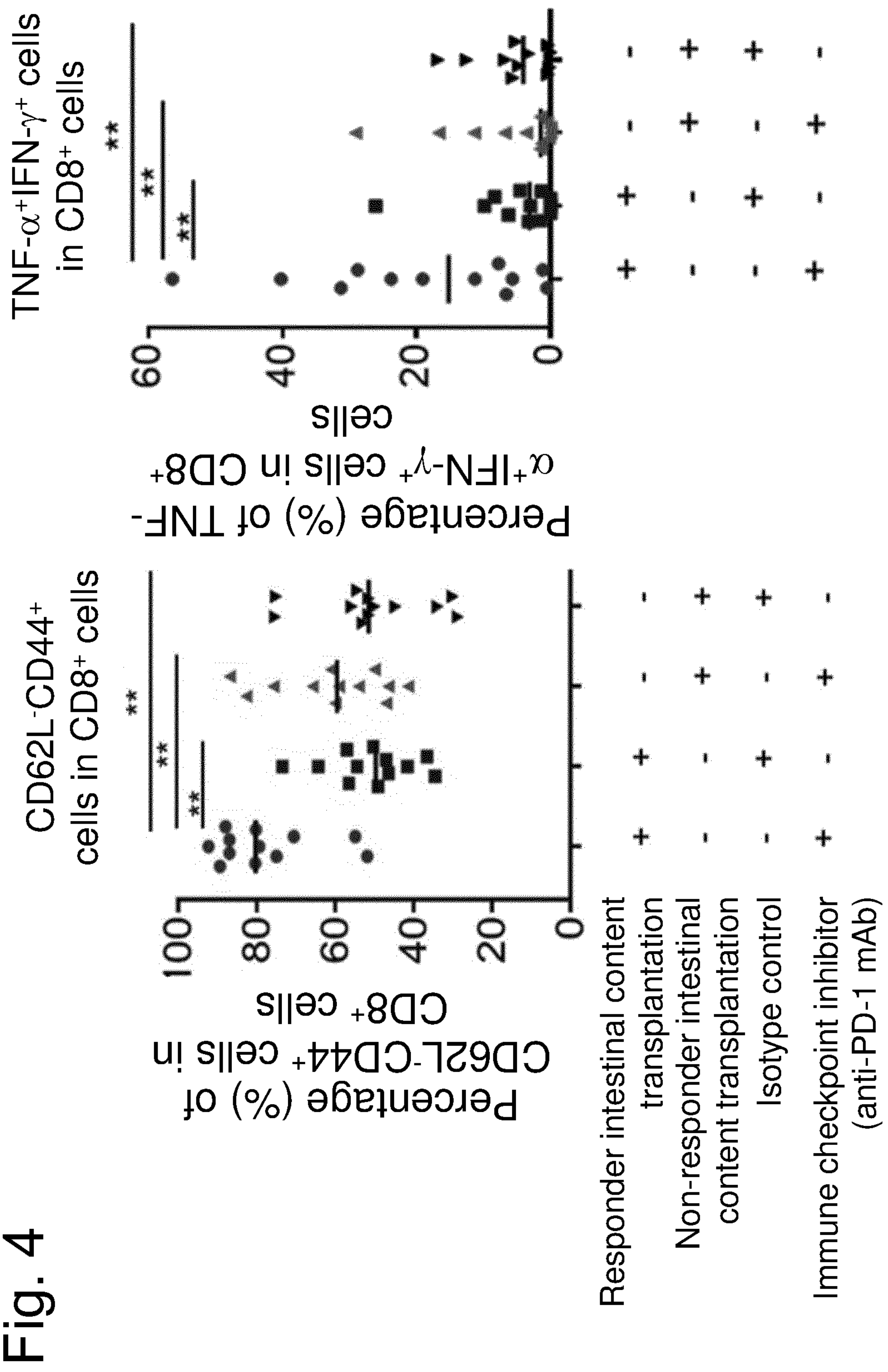
CD62L-CD44+ cells in CD8+ cells
Percentage (%) of CD62L-CD44+ cells in CD8+ cells
TNF-α+IFN-γ+ cells in CD8+ cells
Percentage (%) of TNF-α+IFN-γ+ cells in CD8+ cells
**
Responder intestinal content transplantation
Non-responder intestinal content transplantation
Isotype control
Immune checkpoint inhibitor (anti-PD-1 mAb)

Fig. 5

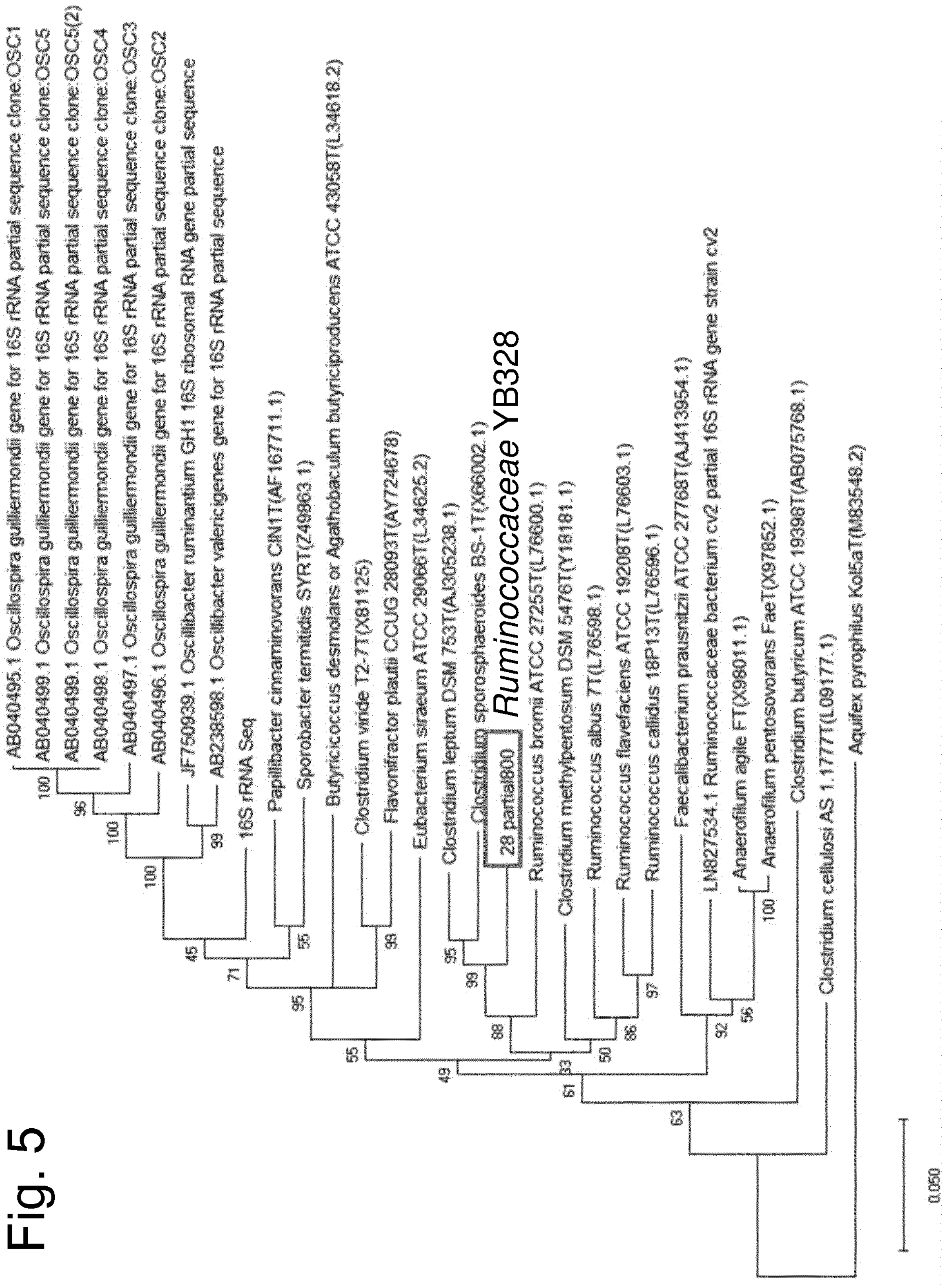

AB040495.1 Oscillospira guilliermondii gene for 16S rRNA partial sequence clone:OSC1
AB040499.1 Oscillospira guilliermondii gene for 16S rRNA partial sequence clone:OSC5
AB040499.1 Oscillospira guilliermondii gene for 16S rRNA partial sequence clone:OSC5(2)
AB040498.1 Oscillospira guilliermondii gene for 16S rRNA partial sequence clone:OSC4
AB040497.1 Oscillospira guilliermondii gene for 16S rRNA partial sequence clone:OSC3
AB040496.1 Oscillospira guilliermondii gene for 16S rRNA partial sequence clone:OSC2
JF750939.1 Oscillibacter ruminantium GH1 16S ribosomal RNA gene partial sequence
AB238598.1 Oscillibacter valericigenes gene for 16S rRNA partial sequence
16S rRNA Seq
Papillibacter cinnaminovorans CIN1T(AF167711.1)
Sporobacter termitidis SYRT(Z49863.1)
Butyricicoccus desmolans or Agathobaculum butyriciproducens ATCC 43058T(L34618.2)
Clostridium viride T2-7T(X81125)
Flavonifractor plautii CCUG 28093T(AY724678)
Eubacterium siraeum ATCC 29066T(L34625.2)
Clostridium leptum DSM 753T(AJ305238.1)
Clostridium sporosphaeroides BS-1T(X66002.1)
28 partial800
Ruminococcaceae YB328
Ruminococcus bromii ATCC 27255T(L76600.1)
Clostridium methylpentosum DSM 5476T(Y18181.1)
Ruminococcus albus 7T(L76598.1)
Ruminococcus flavefaciens ATCC 19208T(L76603.1)
Ruminococcus callidus 18P13T(L76596.1)
Faecalibacterium prausnitzii ATCC 27768T(AJ413954.1)
LN827534.1 Ruminococcaceae bacterium cv2 partial 16S rRNA gene strain cv2
Anaerofilum agile FT(X98011.1)
Anaerofilum pentosovorans FaeT(X97852.1)
Clostridium butyricum ATCC 19398T(AB075768.1)
Clostridium cellulosi AS 1.1777T(L09177.1)
Aquifex pyrophilus Kol5aT(M83548.2)
100
96
100
100
99
45
71
55
95
99
55
95
99
88
49
33
50
86
97
61
92
56
100
63
0.050

Fig. 6

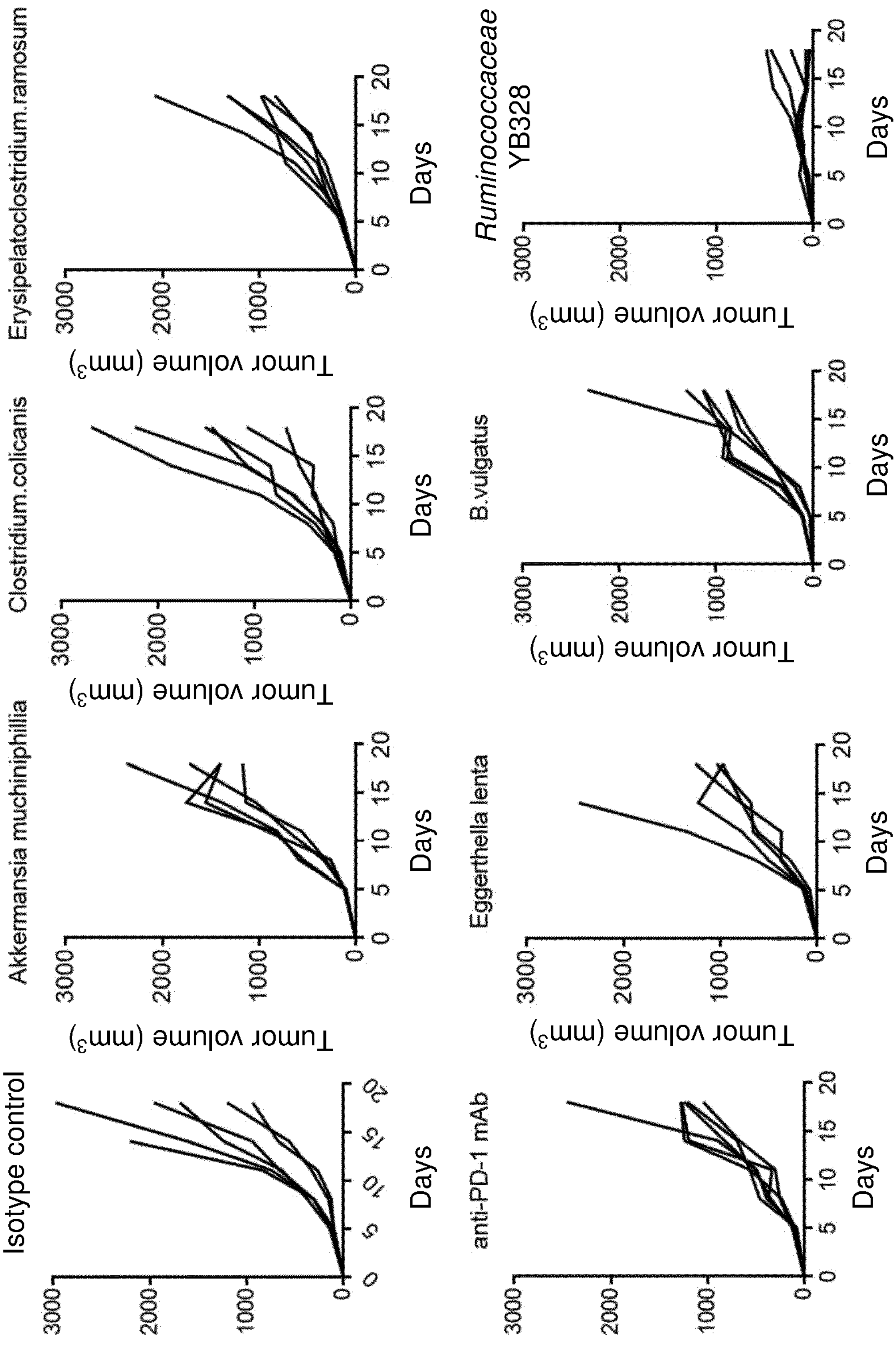
Isotype control
Akkermansia muchiniphillia
Clostridium.colicanis
Erysipelatoclostridium.ramosum
anti-PD-1 mAb
Eggerthella lenta
B.vulgatus
*Ruminococcaceae*
YB328
Tumor volume (mm$^3$)
Days
0
1000
2000
3000
0
5
10
15
20

Fig. 7

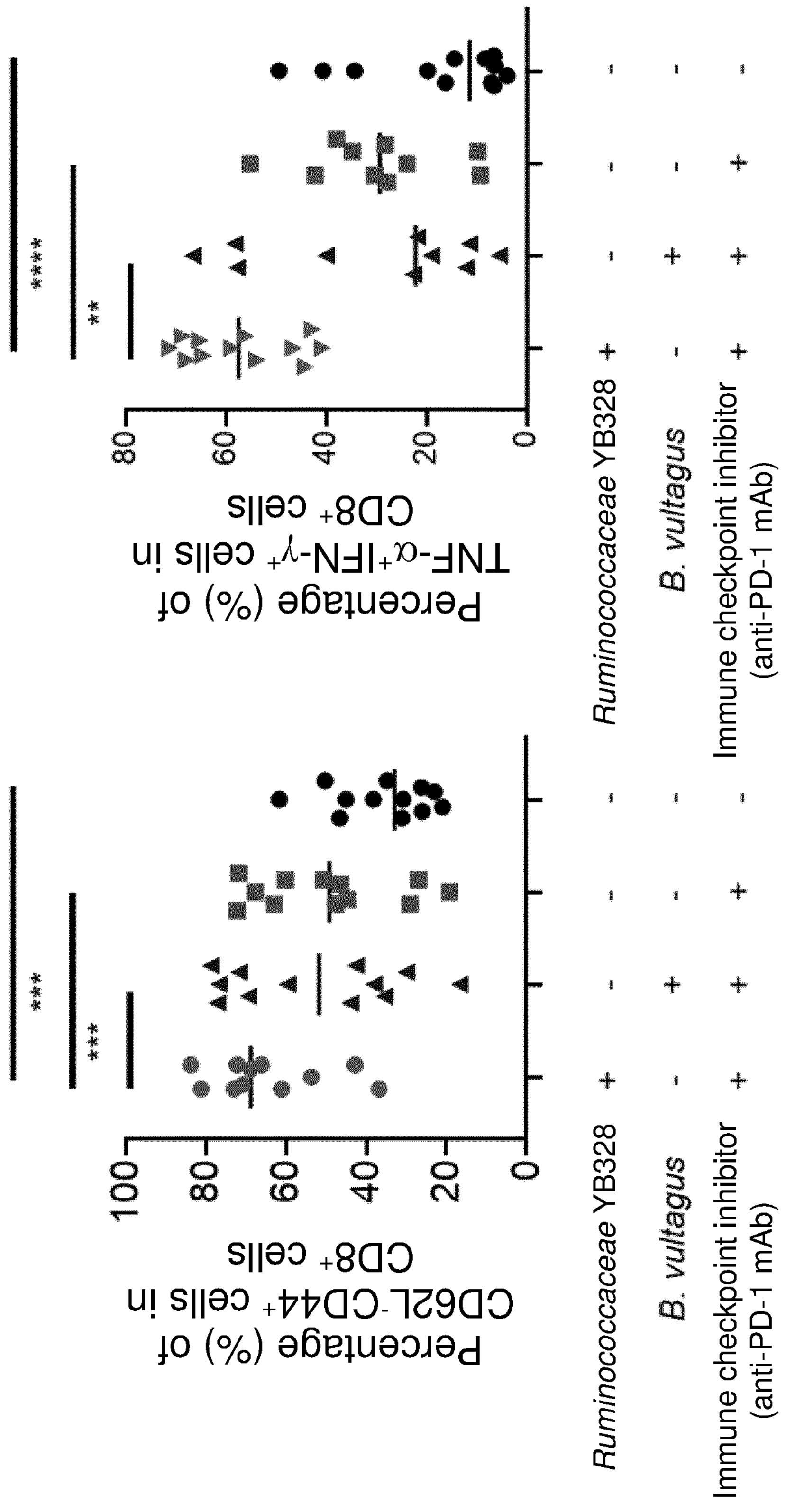
Percentage (%) of CD62L-CD44+ cells in CD8+ cells
100
80
60
40
20
0
***
***
****
Percentage (%) of TNF-α+IFN-γ+ cells in CD8+ cells
80
60
40
20
0
**
****
****
Ruminococcaceae YB328
+
-
-
-
B. vultagus
-
+
-
-
Immune checkpoint inhibitor (anti-PD-1 mAb)
+
+
+
-

Fig. 8

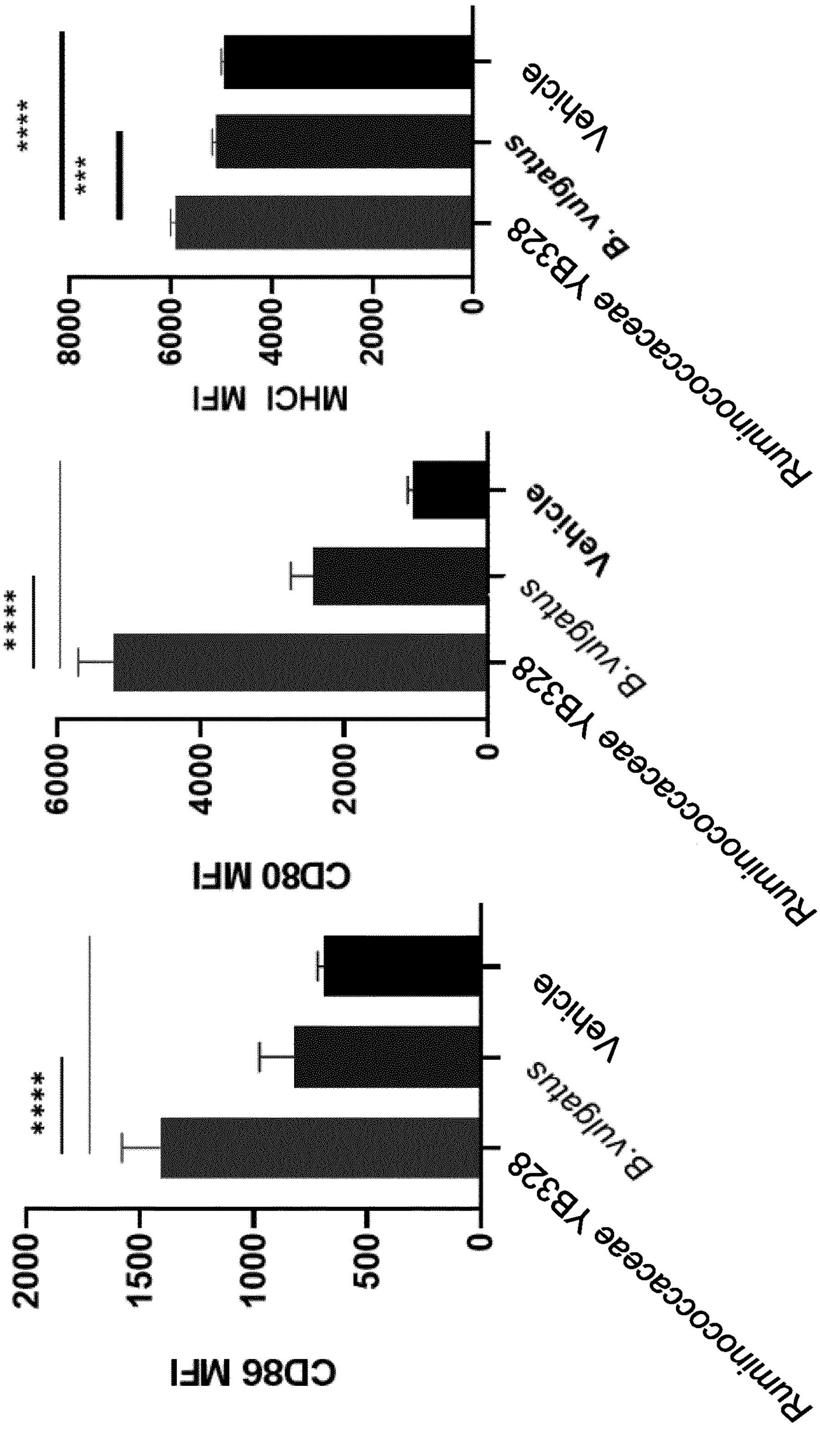
MHC Class I
MHCI MFI
8000
6000
4000
2000
0
****
***
CD80
CD80 MFI
6000
4000
2000
0
****
CD86
CD86 MFI
2000
1500
1000
500
0
****
Ruminococcaceae YB328
B. vulgatus
Vehicle

Fig. 9

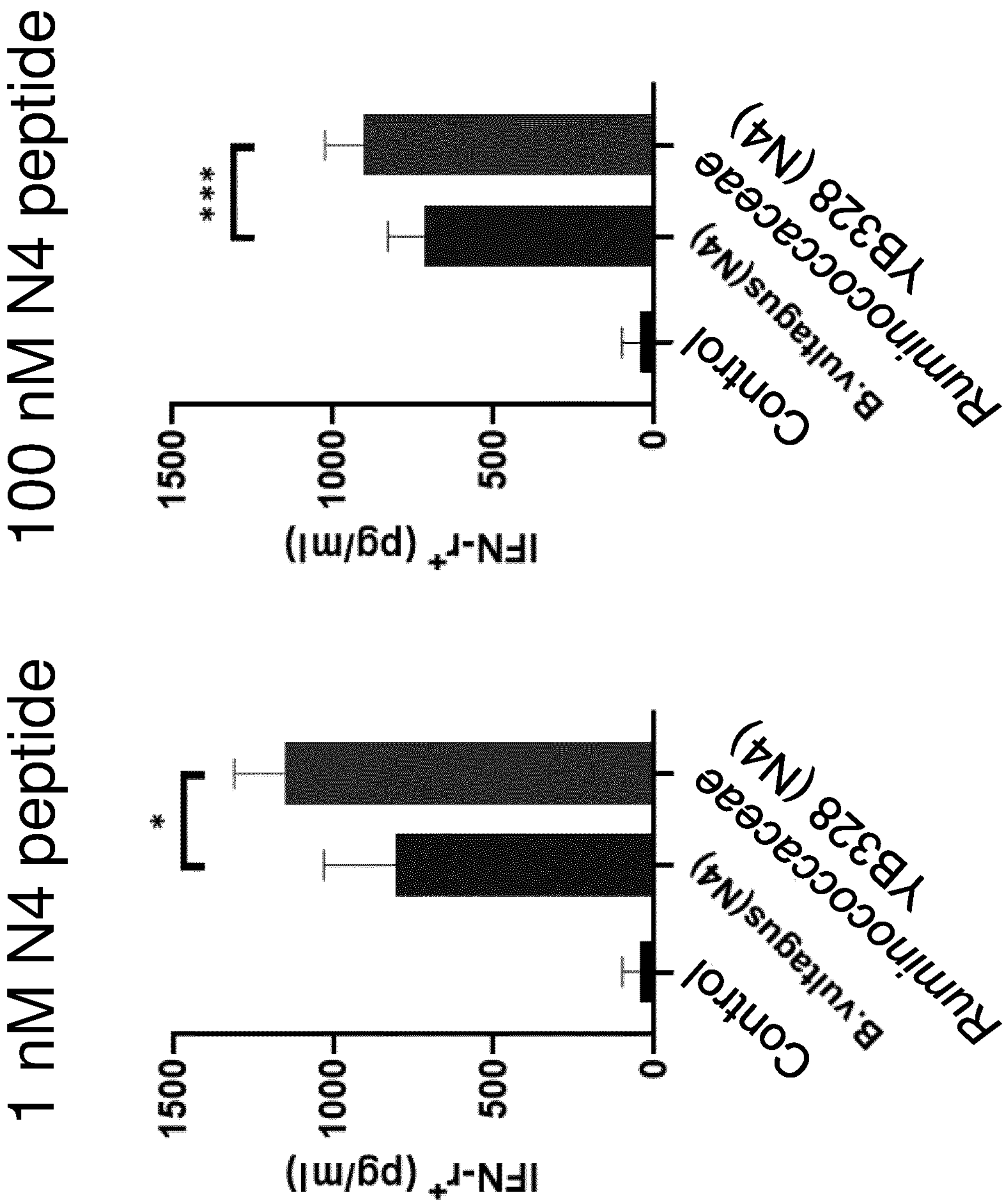
1 nM N4 peptide
100 nM N4 peptide
IFN-r+ (pg/ml)
0
500
1000
1500
*
***
Control
B.vultagus(N4)
Ruminococcaceae YB328 (N4)

Fig. 10

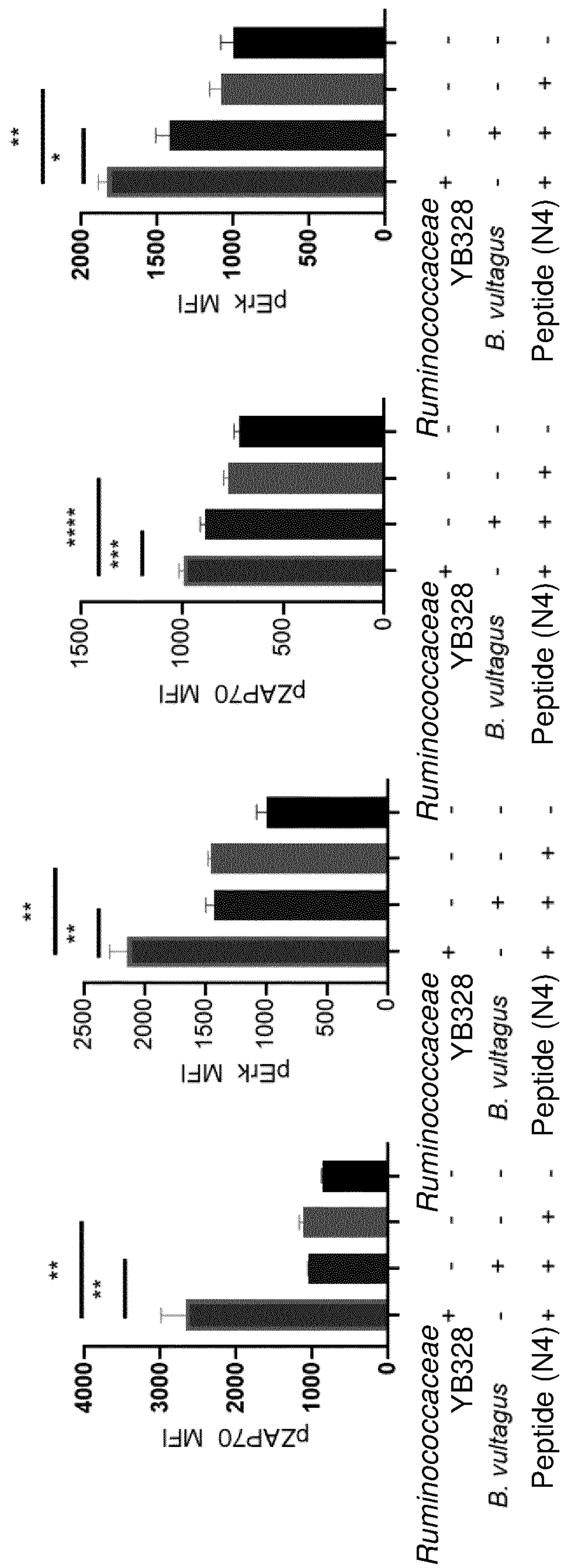
pZAP70 MFI
pErk MFI
pZAP70 MFI
pErk MFI
4000
3000
2000
1000
0
2500
2000
1500
1000
500
0
1500
1000
500
0
2000
1500
1000
500
0
**
***
****
*
Ruminococcaceae YB328
B. vultagus
Peptide (N4)

Fig. 11

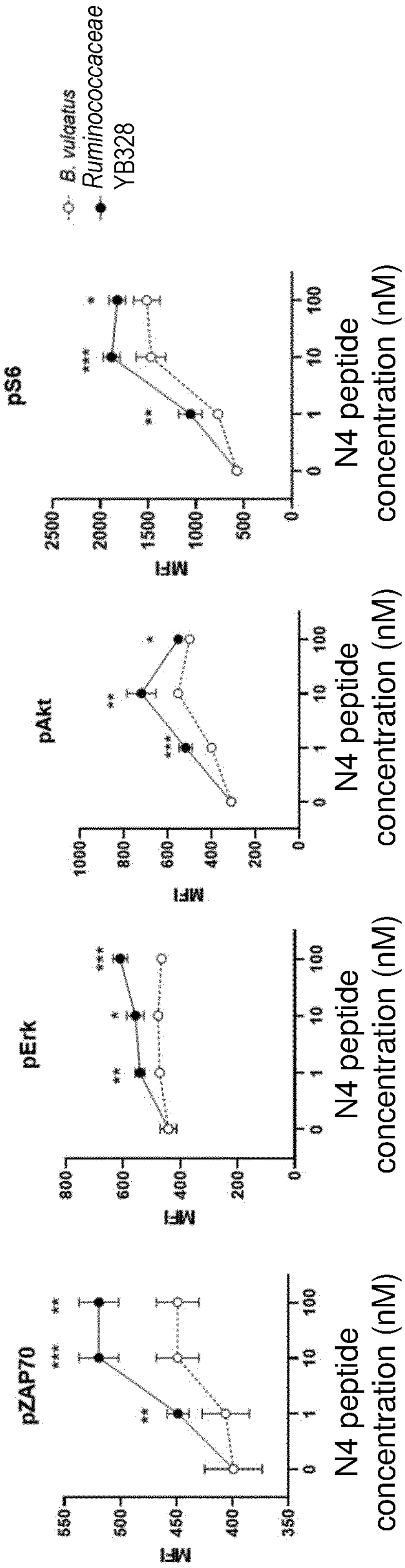
pZAP70
pErk
pAkt
pS6
MFI
N4 peptide concentration (nM)
B. vulgatus
Ruminococcaceae YB328

Fig. 12

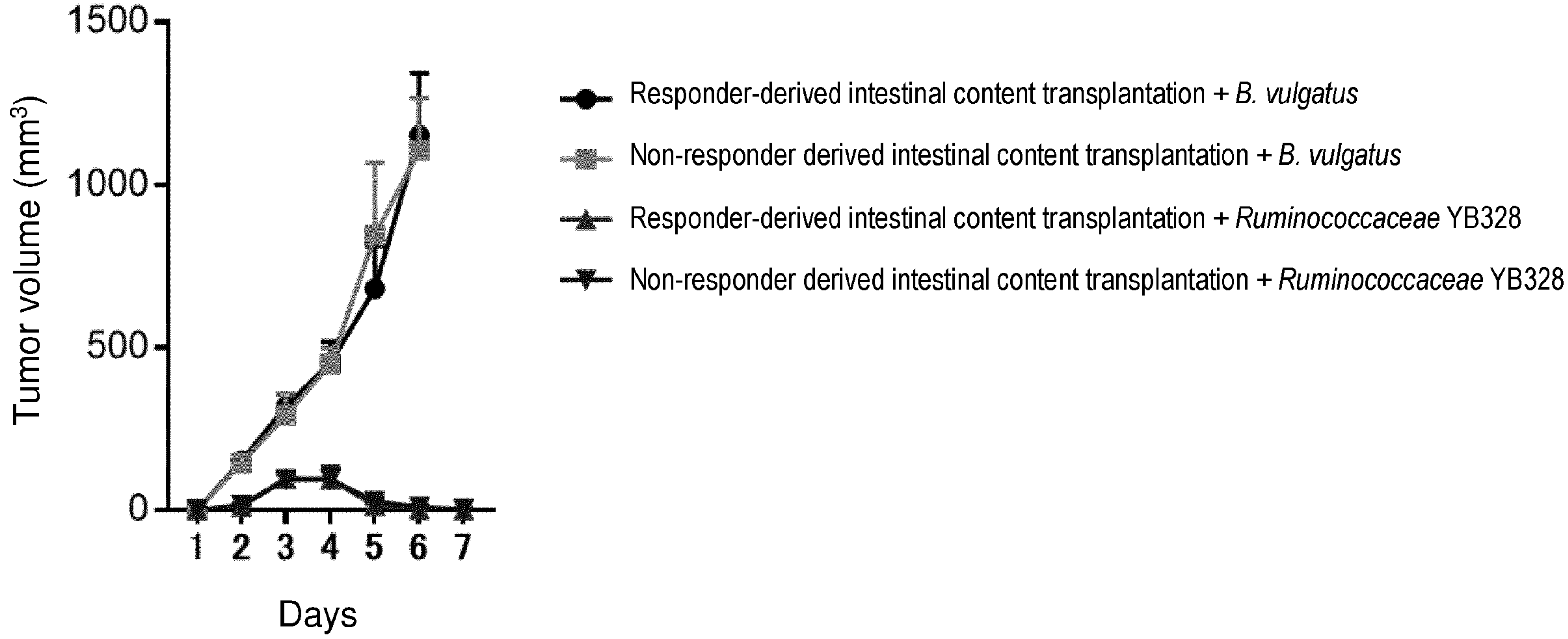
Tumor volume (mm3)
1500
1000
500
0
1 2 3 4 5 6 7
Days
Responder-derived intestinal content transplantation + B. vulgatus
Non-responder derived intestinal content transplantation + B. vulgatus
Responder-derived intestinal content transplantation + Ruminococcaceae YB328
Non-responder derived intestinal content transplantation + Ruminococcaceae YB328

Fig. 13

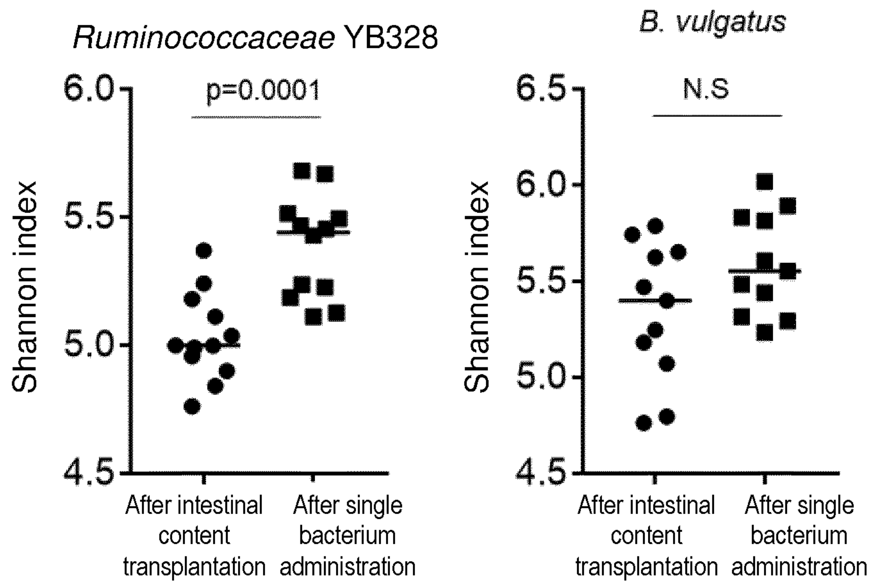
*Ruminococcaceae* YB328
p=0.0001
Shannon index
6.0
5.5
5.0
4.5
After intestinal content transplantation
After single bacterium administration
*B. vulgatus*
N.S
Shannon index
6.5
6.0
5.5
5.0
4.5
After intestinal content transplantation
After single bacterium administration

Fig. 14

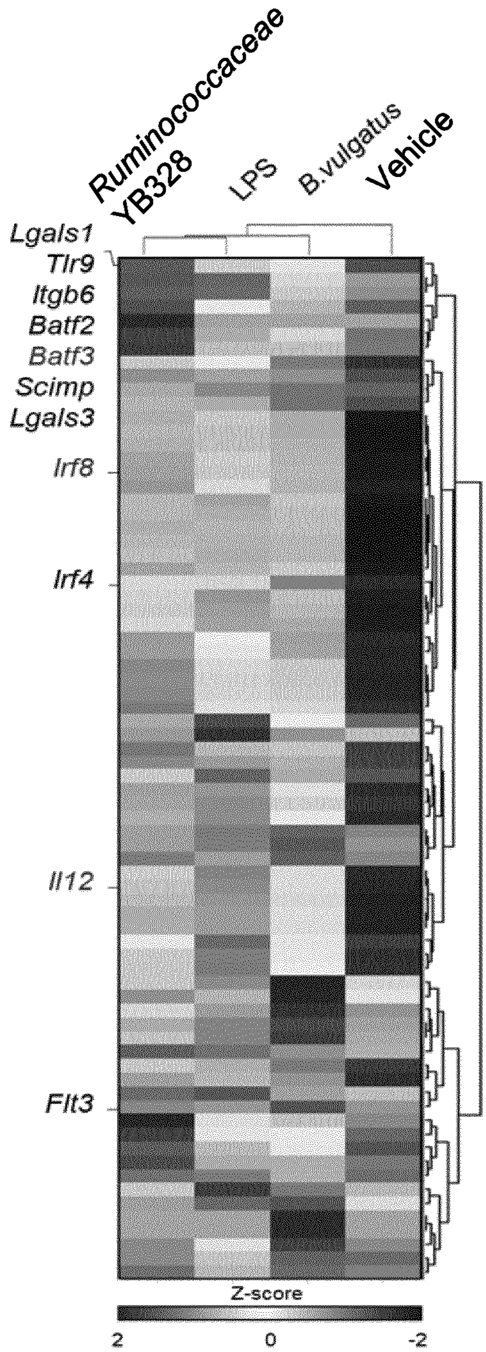
Ruminococcaceae YB328
LPS
B.vulgatus
Vehicle
Lgals1
Tlr9
Itgb6
Batf2
Batf3
Scimp
Lgals3
Irf8
Irf4
Il12
Flt3
Z-score
2
0
-2

Fig. 15

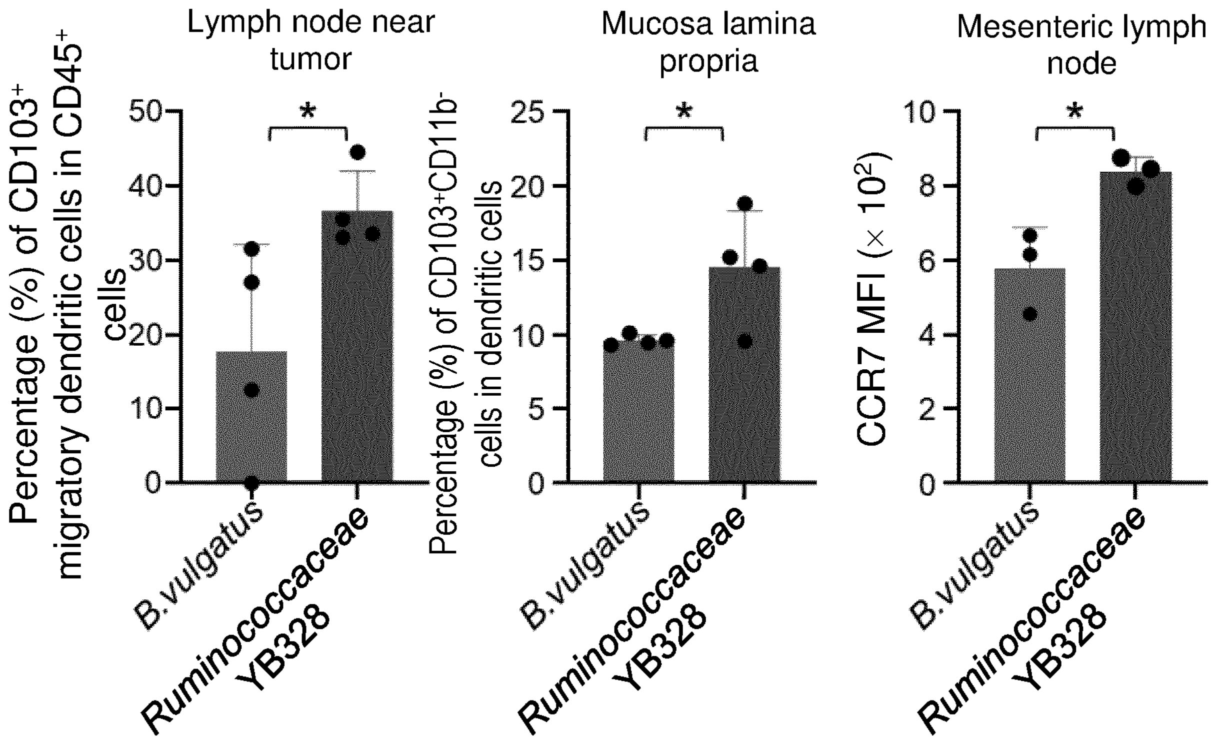

Lymph node near tumor
Percentage (%) of CD103+ migratory dendritic cells in CD45+ cells
50
40
30
20
10
0
*
B.vulgatus
Ruminococcaceae YB328
Mucosa lamina propria
Percentage (%) of CD103+CD11b- cells in dendritic cells
25
20
15
10
5
0
*
B.vulgatus
Ruminococcaceae YB328
Mesenteric lymph node
CCR7 MFI (× 10²)
10
8
6
4
2
0
*
B.vulgatus
Ruminococcaceae YB328

Fig. 16

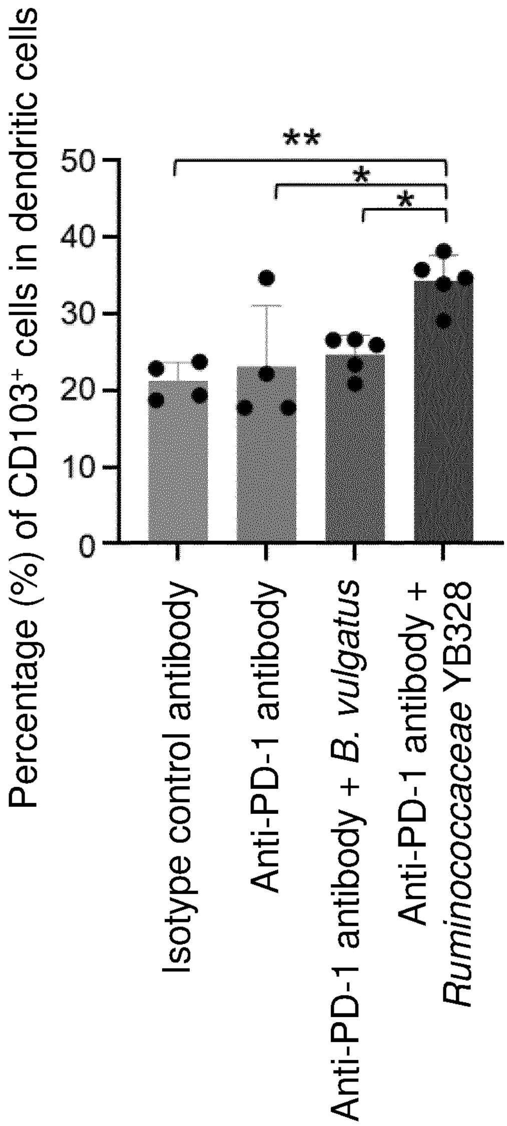
Percentage (%) of CD103+ cells in dendritic cells
50
40
30
20
10
0
**
*
*
Isotype control antibody
Anti-PD-1 antibody
Anti-PD-1 antibody + B. vulgatus
Anti-PD-1 antibody + Ruminococcaceae YB328

Fig. 17

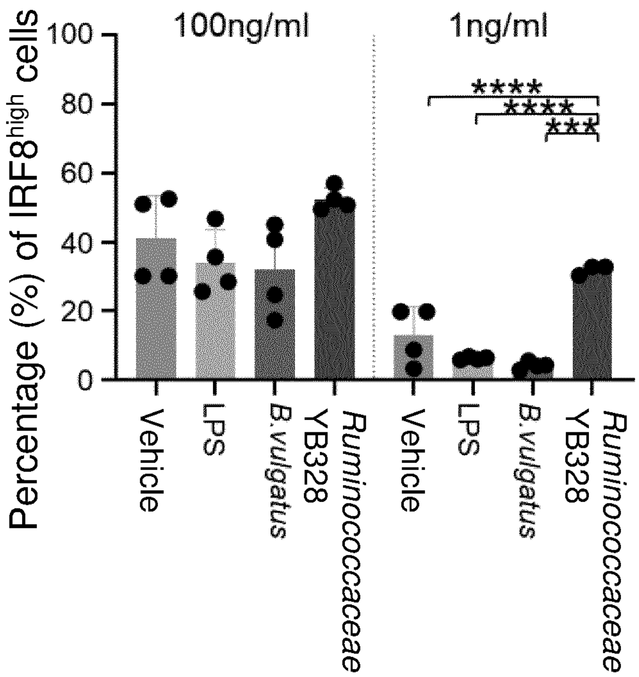
100ng/ml
1ng/ml
Percentage (%) of IRF8$^{high}$ cells
100
80
60
40
20
0
****
****
***
Vehicle
LPS
B.vulgatus
YB328
Ruminococcaceae
Vehicle
LPS
B.vulgatus
YB328
Ruminococcaceae

Fig. 18

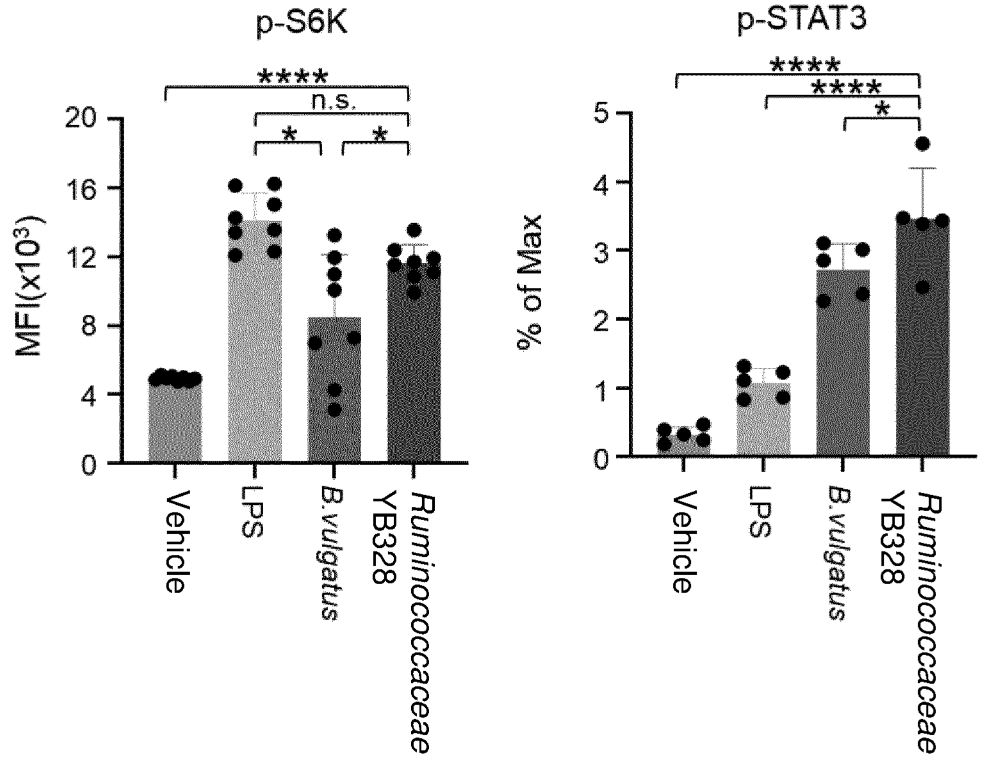
p-S6K
MFI(x10$^3$)
20
16
12
8
4
0
****
n.s.
*
*
Vehicle
LPS
B.vulgatus
YB328
Ruminococcaceae
p-STAT3
% of Max
5
4
3
2
1
0
****
****
*
Vehicle
LPS
B.vulgatus
YB328
Ruminococcaceae

Fig. 19

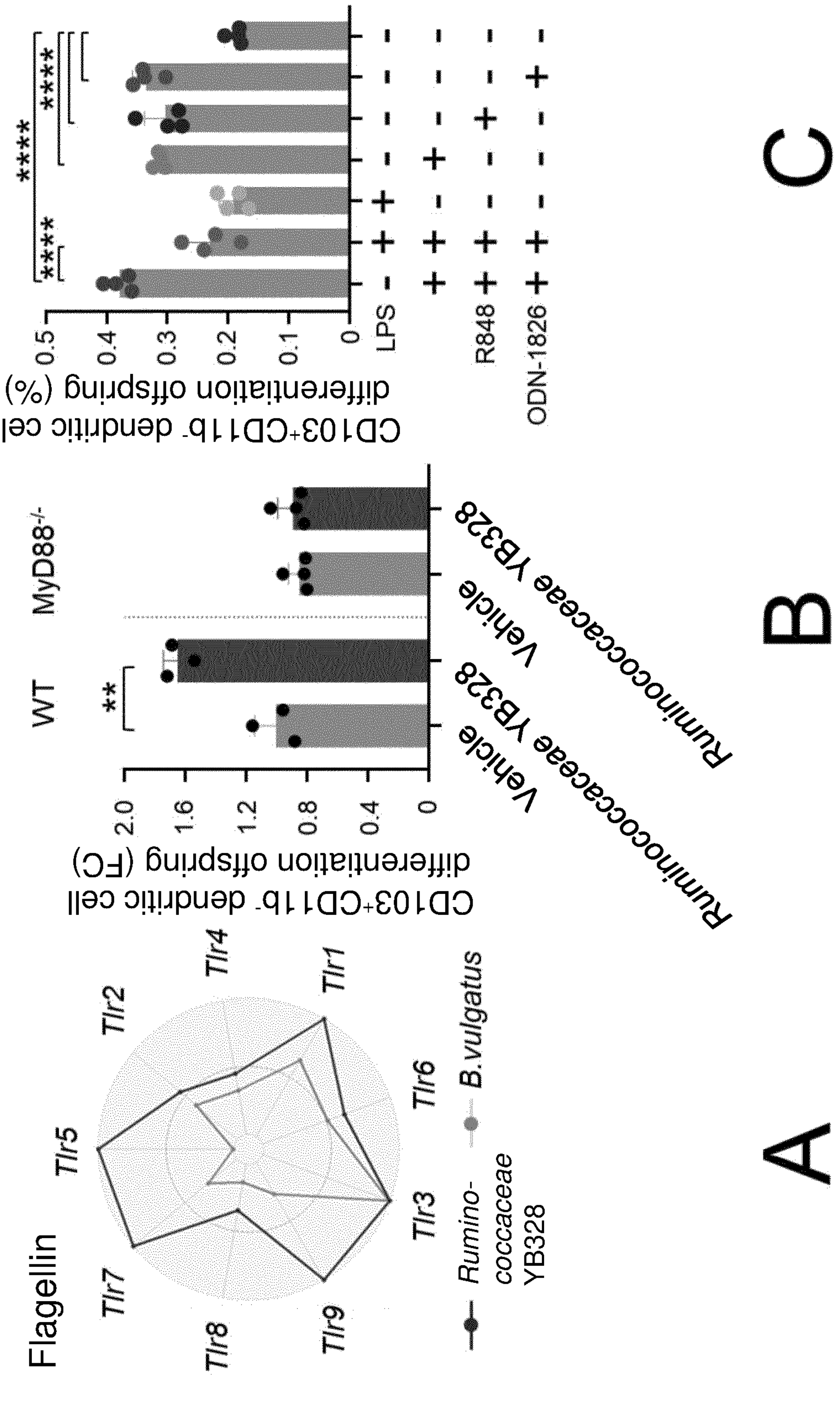
Flagellin
Tlr5
Tlr2
Tlr4
Tlr1
Tlr6
Tlr3
Tlr9
Tlr8
Tlr7
Rumino-coccaceae YB328
B.vulgatus
A
CD103+CD11b- dendritic cell differentiation offspring (FC)
WT
MyD88-/-
**
Vehicle
Ruminococcaceae YB328
B
CD103+CD11b- dendritic cell differentiation offspring (%)
****
****
****
LPS
R848
ODN-1826
C

**INTERNATIONAL SEARCH REPORT**

International application No. **PCT/JP2021/035941**

**A. CLASSIFICATION OF SUBJECT MATTER**

***C12N 1/20***(2006.01)i; ***A61K 35/741***(2015.01)i; ***A61K 39/395***(2006.01)i; ***A61K 45/00***(2006.01)i; ***A61P 35/00***(2006.01)i; ***A61P 35/02***(2006.01)i; ***A61P 35/04***(2006.01)i; ***A61P 37/04***(2006.01)i; ***A61P 43/00***(2006.01)i; ***C12N 15/11***(2006.01)i; ***C12Q 1/6869***(2018.01)i

FI: C12N1/20 A; A61K35/741; A61P43/00 121; A61P35/00; A61P35/02; A61P35/04; A61P37/04; A61K45/00; A61K39/395 U; C12Q1/6869 Z; C12N15/11 Z ZNA

According to International Patent Classification (IPC) or to both national classification and IPC

**B. FIELDS SEARCHED**

Minimum documentation searched (classification system followed by classification symbols)

C12N1/20; A61K35/741; A61K39/395; A61K45/00; A61P35/00; A61P35/02; A61P35/04; A61P37/04; A61P43/00; C12N15/11; C12Q1/6869

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| X | WO 2019/032572 A1 (FINCH THERAPEUTICS, INC.) 14 February 2019 (2019-02-14) claims, page 63, line 23 to page 64, line 2, page 95, line 34 to page 96, line 2 | 6-29 |
| Y | | 1-31, 33-34 |
| A | | 32, 35-37 |
| X | JP 2020-500151 A (BOARD OF REGENTS, THE UNIVERSITY OF TEXAS SYSTEM) 09 January 2020 (2020-01-09) claims, examples | 9-10, 12-31, 33-34 |
| Y | | 1-31, 33-34 |
| A | | 32, 35-37 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 October 2021** | **09 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/035941**

**C. DOCUMENTS CONSIDERED TO BE RELEVANT**

| Category* | Citation of document, with indication, where appropriate, of the relevant passages | Relevant to claim No. |
|---|---|---|
| X | WO 2019/191390 A2 (SERES THERAPEUTICS, INC.) 03 October 2019 (2019-10-03) claims, paragraph [0139], examples | 9-10, 12-31, 33-34 |
| Y | | 1-31, 33-34 |
| A | | 32, 35-37 |
| X | WO 2019/169160 A1 (EVELO BIOSCIENCES, INC.) 06 September 2019 (2019-09-06) claims, paragraph [0133], examples | 9-10, 12-31, 33-34 |
| Y | | 1-31, 33-34 |
| A | | 32, 35-37 |
| X | WO 2018/226690 A1 (THE UNIVERSITY OF CHICAGO) 13 December 2018 (2018-12-13) claims, page 7, line 22 to page 8, line 23, examples | 9-10, 12-31, 33-34 |
| Y | | 1-31, 33-34 |
| A | | 32, 35-37 |
| X | JP 2020-512294 A (INSTITUT GUSTAVE ROUSSY) 23 April 2020 (2020-04-23) claims, paragraphs [0086]-[0088], examples | 9-10, 12-31, 33-34 |
| Y | | 1-31, 33-34 |
| A | | 32, 35-37 |
| Y | Database GenBank [online], Accession No. GQ897738.1, 09 March 2010 updated [retrieved on 27 October 2021], Definition: Uncultured bacterium clone A3-21 16S ribosomal RNA gene. partial sequence. <URL: http://www.ncbi.nlm.nih.gov/nuccore/261261887?sat=4&satkey=38930936> entire text | 1-31, 33-34 |
| A | | 32, 35-37 |
| X | SPRANGER, S. et al. Generation of Th1-Polarizing Dendritic Cells Using the TLR7/8 Agonist CL075. J. Immunol. 2010, 185(1), pp. 738-747 abstract, page 746, left column, second paragraph | 30, 34-37 |
| A | | 1-29, 31-33 |
| X | JP 2013-522309 A (THE TRUSTEES OF THE UNIVERSITY OF PENNSYLVANIA) 13 June 2013 (2013-06-13) paragraphs [0086]-[0089] | 30-32, 34-37 |
| A | | 1-29, 33 |

Form PCT/ISA/210 (second sheet) (January 2015)

**INTERNATIONAL SEARCH REPORT**

International application No.
**PCT/JP2021/035941**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/JP2021/035941**

| Patent document cited in search report | Publication date (day/month/year) | Patent family member(s) | Publication date (day/month/year) |
|---|---|---|---|
| WO 2019/032572 A1 | 14 February 2019 | US 2020/0093870 A1 | |
| | | EP 3665181 A1 | |
| | | KR 10-2020-0037851 A | |
| | | CA 3072029 A | |
| | | AU 2018313765 A | |
| | | CN 111328331 A | |
| JP 2020-500151 A | 09 January 2020 | US 2020/0129569 A1 claims, examples | |
| | | WO 2018/064165 A2 | |
| | | EP 3518946 A2 | |
| | | CA 3038076 A | |
| | | AU 2017335732 A | |
| | | KR 10-2019-0061042 A | |
| | | BR 112019006041 A | |
| | | CN 110267651 A | |
| | | MX 2019003447 A | |
| WO 2019/191390 A2 | 03 October 2019 | EP 3773915 A2 | |
| WO 2019/169160 A1 | 06 September 2019 | US 2021/0052669 A1 | |
| | | WO 2019/152667 A1 | |
| | | EP 3746121 A1 | |
| WO 2018/226690 A1 | 13 December 2018 | US 2021/0177918 A1 | |
| | | EP 3634443 A1 | |
| JP 2020-512294 A | 23 April 2020 | WO 2018/115519 A1 claims, page 42, line 29 to page 43, line 23, examples | |
| | | EP 3559257 A1 | |
| | | CA 3047029 A | |
| | | AU 2017380257 A | |
| | | IL 267564 D | |
| | | KR 10-2019-0118151 A | |
| | | CN 110582291 A | |
| JP 2013-522309 A | 13 June 2013 | US 2013/0183343 A1 paragraphs [0093]-[0096] | |
| | | WO 2011/115970 A1 | |
| | | EP 2547360 A1 | |
| | | EP 3424522 A1 | |
| | | AU 2011227447 A | |
| | | CA 2793458 A | |
| | | CN 102933228 A | |
| | | RU 2012143745 A | |
| | | BR 112012023285 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015518826 A


### Non-patent literature cited in the description


- *Nature Reviews Cancer,* 2012, vol. 12, 252-264
- *Science,* 2018, vol. 359, 91-97
- *Science,* 2018, vol. 359, 97-103
- **TOMOTARI MITSUOKA.** The World of Enterobacteria. Asakura Publishing Co., Ltd, 1990
- **DEVEREUX et al.** *Nucl. Acids Res,* 1984, vol. 12, 387